# EUROPEAN PATENT APPLICATION

(11) **EP 1 782 829 A1**
(43) Date of publication of application: **09.05.2007**
(21) Application number: 05770532.9
(22) Date of filing: 09.08.2005
(51) Int. Cl.: A61K 45/00, A61K 31/5575, A61K 38/28, A61K 45/06, A61K 47/40, A61P 1/10, A61P 1/12, A61P 3/02, A61P 3/10

(54) **PREVENTIVE AND/OR REMEDY FOR HYPERKALEMIA CONTAINING EP4 AGONIST**

(30) Priority: 10.08.2004 JP 2004232984
(71) Applicant: ONO PHARMACEUTICAL CO., LTD., Osaka-shi, Osaka 541-8526 (JP)
(72) Inventor: KUWAHARA, Atsukazu, Shizuoka-shi, Shizuoka 422-8002 (JP); SUZUKI, Yuichi, Shizuoka-shi, Shizuoka 422-8002 (JP); MARUYAMA, Takayuki, c/o Ono Pharmaceutical Co. Ltd, Mishima-gun, Osaka 618-8585 (JP)
(74) Representative: Henkel, Feiler & Hänzel
(86) International application number: PCT/JP2005/014885
(87) International publication number: WO 2006/016695

(57) **Abstract**

The present invention relates to a preventive and/or therapeutic agent for hyperkalemia, and a potassium excretion promoter containing EP₄ agonist. Since EP₄ agonist promotes potassium excretion, it is useful as a preventive and/or therapeutic agent for hyperkalemia. In addition, if selective EP₄ agonist uses, it is a preventive and/or therapeutic agent for hyperkalemia without side effects. Further, if EP₄ agonist is used, it is useful as improving agent for various symptoms (e.g. paresthesia, error of perception, weakness, myoparalysis, nausea, vomit, abdominal pain, diarrhea, arrhythmia, atrioventricular block, ventricular fibrillation, atrial fibrillation, cardiac arrest, asphyxia and/or dyspnoea *etc*.).

## Description

### Technical Field

The present invention relates to (1) a preventive and/or therapeutic agent for hyperkalemia, (2) a potassium excretion promoter and (3) a serum potassium concentration lowering agent, containing EP₄ agonist useful for medical drug.

### Background Art

Hyperkalemia is a disease which is caused by excess serum potassium above normal. Normal value of serum potassium concentration in human is generally in the range of 3.5 to 5.0 mEq/l. When this concentration is above 5.5 mEq/l, it diagnoses hyperkalemia.

Such hyperkalemia has causes of, for example, (1) the potassium loads increase by consuming foods containing of much potassium in excess and the like, (2) the flow amounts of potassium from intracellular to extracellular increase caused by (a) acidosis (particularly respiratory acidosis), (b) shortage of caloric intake, (c) prexia and infectious disease, (d) wasting illness, (e) hemolysis in blood vessels, (f) gastrointestinal bleeding, (g) insulin deficiency, (h) administration of beta blocker and the like, (3) potassium excretion defect from kidney is caused by (a) decreased kidney function by kidney impairment such as, renal insufficiency, nephritis, graft rejection by renal transplantation, uropathy and the like, (b) decreased aldosterone which is a hormone modulating an excretion of sodium and potassium in kidney, (c) disorder in potassium excretion from kidney caused by side effects by medication (*e.g.*, angiotensin converting enzyme inhibitors, angiotensin II receptor antagonists, nonsteroidal antiinflammatory drugs, potassium sparing diuretics, potassium supplements *etc*.) to patients affected by kidney impairment and the like. Especially, it is said that its main cause is disorder in potassium excretion from kidney.

Symptoms of hyperkalemia list, for example, (1) neural and/or muscular disorder such as, paresthesia, error of perception, weakness, myoparalysis (*e.g.*, dyspnea caused by diaphragmatic paralysis, asphyxia *etc*.) and the like, (2) gastrointestinal dysfunction such as, nausea, vomit, abdominal pain, diarrhea and the like, (3) circulatory dysfunction such as, feeble heart sound, electrocardiogram change, arrhythmia, ventricular fibrillation, atrial fibrillation, atrioventricular block, cardiac arrest and the like. These symptoms are from slight ones to severe ones and the therapeutic methods vary depending on their stage. As an acute therapeutic method, for example, (a) in case of cadioplesia or arrhythmia, temporarily transvenous administration of calcium, (b) transvenous administration of glucose or insulin, (c) in case of acidosis, administration of sodium bicarbonate, (d) administration of diuretics, (e) oral administration or intestinal administration of cation exchange resin (e,g., sodium polystyrene sulfonate, calcium polystyrene sulfonate *etc*.), (f) hemodialysis and the like are performed. In contrast, as a long-term therapeutic method, diet therapy decreasing potassium intake on diet is performed.

It is reported that prostanoic acid derivatives having oxo group at 15th position are useful as a preventive and/or therapeutic agent for such a hyperkalemia (refer to EP410652). It is also reported that prostanoic acid derivatives having oxo group at 15th position are useful as a uterine contraction agent and a laxative (refer to EP342003 or EP310305). Moreover, it is reported that EP₄ agonists are useful as a preventive agent and/or therapeutic agent for renal failure or renal dysfunction (refer to EP1132086). However, there are no descriptions about hyperkalemia.

It is noted that in the colon, PGE₂ induced potassium ion secretion at low concentration and predominantly induced chlorine ion secretion at high concentration (refer to American Journal of Physiology Gastrointestinal Liver Physiology, vol. 281, pp. G984, (2001) or American Journal of Physiology Gastrointestinal Liver Physiology, vol. 283, pp. G347, (2002)). However, it is not noted that EP₄ is involved in excreting potassium externally.

### Disclosure of the Invention

Since the patients affected with renal failure or renal dysfunction decrease the function excreting potassium from urine, their potassium concentration in blood elevates to cause hyperkalemia. Hyperkalemia advances severely to lead to death in some case. It is hoped that an effective therapeutic agent for it is developed, however, a useful therapeutic agent for it is not found out yet at present.

In addition, prostanoic acid derivatives having oxo group at 15th position which are known to therapeutic agents for hyperkalemia have various pharmacological actions. They are known to, for example, elevated heart rate, elevated blood pressure, uterine contraction, myocardial contraction, diarrhea, temperature rise and the like. However, since they have such various pharmacological properties, the above described actions act as side effects in case of use to patients affected with hyperkalemia.

Moreover, since PGE₂ predominantly induces chlorine ion secretion at high concentration and this chlorine ion secretion accompanies water secretion, it relates to severe diarrhea. Accordingly, when PGE₂ is used for the patients affected with hyperkalemia at high concentration, diarrhea is observed as a side effect.

Accordingly, the development of a free of side-effect and safe preventive and/or therapeutic agent for hyperkalemia has been longed for. The present inventors conducted intensive investigation, and as a result, found that (1) EP₄ agonists, PGE₂ receptor subtype, stimulate excretion of potassium in blood externally, (2) EP₄ agonists are useful as a preventive and/or therapeutic agent for hyperkalemia, (3) selective EP₄ agonists come to be a preventive and/or therapeutic agent for hyperkalemia without side-effects such as, diarrhea and the like, because they do not cause chlorine ion secretion, and/or (4) EP₄ agonists come to be improving agent for symptoms of hyperkalemia and they have completed the present invention.

That is, the present invention relates to,
1. A preventive and/or therapeutic agent for hyperkalemia comprising EP₄ agonist,
2. The preventive and/or therapeutic agent according to the above 1, wherein one or more symptom(s) selected from nerve abnormality, muscular abnormality, circulatory abnormality and digestive abnormality is/are improved,
3. The preventive and/or therapeutic agent according to the above 1, wherein one or more symptom(s) selected from arrhythmia, atrioventricular block, ventricular fibrillation, atrial fibrillation and dyspnoea is/are improved,
4. A potassium excretion promoter, comprising EP₄ agonist,
5. The agent according to the above 1 or 4, wherein the EP₄ agonist and one or more agent(s) selected from calcium formulation, glucose formulation, insulin formulation, sodium bicarbonate formulation, diuretic, and cation exchange resin are used in combination,
6. The agent according to the above 1 or 4, wherein the EP₄ agonist is a compound represented by formula (I): 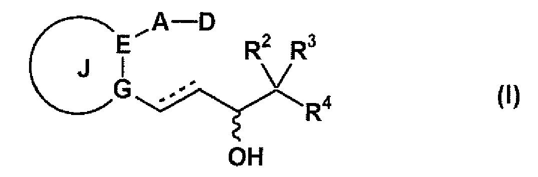 wherein 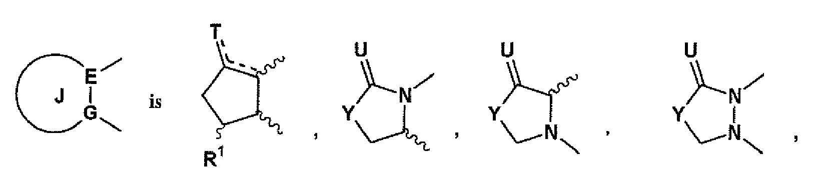 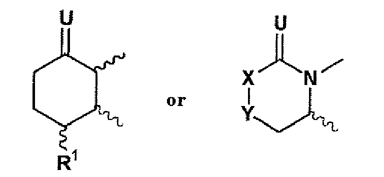 wherein T is an oxygen atom, a halogen atom or acyloxy which may have a substituent(s),
   R¹ is a hydrogen atom, hydroxy, C1-6 alkyloxy or C1-6 acyloxy,
   U is an oxygen atom or a sulfur atom,
   X and Y are each independently methylene, oxygen atom, sulfur atom, or nitrogen atom which may have a substituent(s), wherein X and Y are not an oxygen atom, a sulfur atom nor a nitrogen atom which may have a substituent(s) simultaneously,
   A is a spacer of which main chain has an atom number of 1-8 and which may have a substituent(s),
   D is an acidic group which may be protected,
   R² and R³ are each independently alkyl which may have a substituent(s) or a halogen atom,
   R⁴ is a cyclic group which may have a substituent(s) or aliphatic hydrocarbon which may have a substituent(s),
   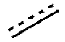 is a single bond or a double bond, however, wherein continuous double bonds are not formed,
   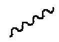 is α-configuration or β-configuration or a mixture thereof having an optical mixing ratio,
   a salt thereof, an N-oxide thereof or a solvate thereof, or a prodrug thereof, or a cyclodextrin clathrate thereof,
7. The agent according to the above 1 or 4, wherein the EP₄ agonist is a compound represented by formula (IA): 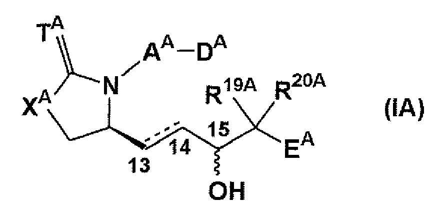 wherein 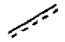 is (1) a single bond or (2) a double bond,
   R^{19A} and R^{20A}are each independently, (1) a hydrogen atom, (2) C1-10 alkyl or (3) a halogen atom,
   T^{A} is (1) an oxygen atom or (2) a sulfur atom,
   X^{A} is (1) -CH₂-, (2) -O- or (3) -S-,
   A^{A} is A^{1A} or A^{2A},
   A^{1A} is (1) C2-8 straight-chain alkylene which may be substituted by 1 to 2 C1-4 alkyl, (2) C2-8 straight-chain alkenylene which may be substituted by 1 to 2 C1-4 alkyl or (3) C2-8 straight-chain alkynylene which may be substituted by 1 to 2 C1-4 alkyl,
   A^{2A} is -G^{1A} -G^{2A} -G^{3A} -,
   G^{1A} is (1) C1-4 straight-chain alkylene which may be substituted by 1 to 2 C1-4 alkyl, (2) C2-4 straight-chain alkenylene which may be substituted by 1 to 2 C1-4 alkyl or (3) C2-4 straight-chain alkynylene which may be substituted by 1 to 2 C1-4 alkyl,
   G^{2A} is (1) -Y^{A} -, (2) -(ring1^{A})-, (3) -Y^{A}-(ring1^{A})-, (4) -(ring1^{A})-Y^{A}- or (5) -Y^{A}-(C1-4 alkylene)-(ring1^{A})-,
   Y^{A} is (1) -S-, (2) -SO-, (3) -SO₂-, (4) -O- or (5) -NR^{1A}-,
   R^{1A} is (1) a hydrogen atom, (2) C1-10 alkyl or (3) C2-10 acyl,
   G^{3A} is (1) a bond, (2) C1-4 straight-chain alkylene which may be substituted by 1 to 2 C1-4 alkyl, (3) C2-4 straight-chain alkenylene which may be substituted by 1 to 2 C1-4 alkyl or (4) C2-4 straight-chain alkynylene which may be substituted by 1 to 2 C1-4 alkyl,
   D^{A} is D^{1A} or D^{2A},
   D^{1A} is (1) -COOH, (2) -COOR^{2A}, (3) tetrazol-5-yl or (4) CONR^{3A}SO₂R^{4A},
   R^{2A} is (1) C1-10 alkyl, (2) phenyl, (3) C1-10 alkyl substituted by phenyl or (4) biphenyl,
   R^{3A} is (1) a hydrogen atom or (2) C1-10 alkyl,
   R^{4A} is (1) C1-10 alkyl or (2) phenyl,
   D^{2A} is (1) -CH₂OH, (2) -CH₂OR^{5A}, (3) hydroxy, (4) -OR^{5A}, (5) formyl, (6) -CONR^{6A}R^{7A}, (7) -CONR^{6A}SO₂R^{8A}, (8) -CO-(NH-amino acid residue-CO)_{mA} -OH, (9) -O-(CO- amino acid residue -NH)_{mA} -H, (10) -COOR^{9A}, (11) -OCO-R^{10A}, (12) -COO-Z^{1A} -Z^{2A} -Z^{3A}, (13) 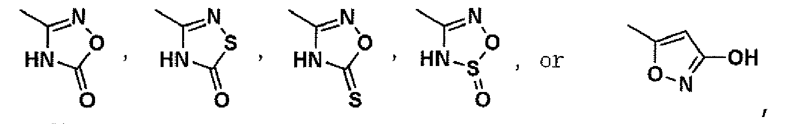
   R^{5A} is C1-10 alkyl,
   R^{6A} and R^{7A} are each independently, (1) a hydrogen atom or (2) C1-10 alkyl,
   R^{8A} is C1-10 alkyl substituted by phenyl,
   R^{9A} is (1) C1-10 alkyl substituted by biphenyl which may be substituted by 1 to 3 C1-10 alkyl, C1-10 alkoxy or halogen atom or (2) biphenyl substituted by 1 to 3 C1-10 alkyl, C1-10 alkoxy or a halogen atom,
   R^{10A} is (1) phenyl or (2) C1-10 alkyl,
   mA is 1 or 2,
   Z^{1A} is (1) C1-15 alkylene, (2) C2-15 alkenylene or (3) C2-15 alkynylene,
   Z^{2A} is (1) -CO-, (2) -OCO-, (3) -COO-, (4) -CONR^{11A}-, (5) -NR^{12A}CO-, (6) -O-, (7) -S-, (8) -SO-, (9) -SO₂-, (10) -NR^{13A}-, (11) -NR^{14A}CONR¹⁵-, (12) -NR^{16A}COO-, (13) -OCONR^{17A}- or (14) -OCOO-,
   Z^{3A} is (1) a hydrogen atom, (2) C1-15 alkyl, (3) C2-15 alkenyl, (4) C2-15 alkynyl, (5) ring2^{A} or (6) C1-10 alkyl substituted by C1-10 alkoxy, C1-10 alkylthio,
   C1-10 alkyl-NR^{18A}- or ring2^{A},
   R^{11A}, R^{12A}, R^{13A}, R^{14A}, R^{15A}, R^{16A}, R^{17A} and R^{18A} are each independently (1) a hydrogen atom or (2) C1-15 alkyl,
   R^{11A} and Z^{3A} may be taken together with the nitrogen atom to which they are attached to form a 5- to 7-membered saturated mono-heterocyclic ring, and the heterocyclic ring may contain other one hetero atom selected from oxygen, nitrogen and sulfur atom(s),
   E^{A} is E^{1A} or E^{2A} ,
   E^{1A} is (1) C3-7 cycloalkyl or (2) ring3^{A},
   E^{2A} is (1) C3-7 cycloalkyl, (2) ring4^{A} or (3) ring5^{A},
   ring1^{A} and ring5^{A} may be substituted by 1 to 3 R^{21A} and/or R^{22A},
   ring3^{A} may be substituted by 1 to 2 R^{21A},
   C3-7 cycloalkyl represented by E^{2A} is substituted by one of R^{21A} or R^{22A}, and may be substituted by another 1 to 2 R^{21A} and/or R^{22A},
   ring4^{A} is substituted by one of R^{22A}, may be substituted by another 1 to 2 R^{21A} and/or R^{22A}, and may be substituted by heterocyclic ring formed by R^{11A},
   Z^{3A} and the nitrogen to which Z^{3A} is attached or ring2^{A} may be substituted by R^{23A},
   R^{21A} is (1) C1-10 alkyl, (2) C1-10 alkoxy, (3) a halogen atom, (4) nitro, (5) C1-10 alkyl substituted by 1 to 3 halogen atom(s) or (6) phenyl,
   R^{22A} is (1) C2-10 alkenyl, (2) C2-10 alkynyl, (3) C1-10 alkylthio, (4) hydroxy, (5) -NR^{24A}R^{25A}, (6) C1-10 alkyl substituted by C1-10 alkoxy, (7) C1-10 alkyl substituted by C1-10 alkoxy substituted by 1 to 3 halogen atom(s), (8) C1-10 alkyl substituted by -NR^{24A}R^{25A}, (9) ring6^{A}, (10) -O-ring7^{A}, (11) C1-10 alkyl substituted by ring7^{A}, (12) C2-10 alkenyl substituted by ring7^{A}, (13) C2-10 alkynyl substituted by ring7^{A}, (14) C1-10 alkoxy substituted by ring7^{A}, (15) C1-10 alkyl substituted by -O-ring7^{A}, (16) -COOR²⁶A or (17) C1-10 alkoxy substituted by 1 to 3 halogen atom(s),
   R^{24A}, R^{25A} and R^{26A} are each independently, (1) a hydrogen atom or (2) C1-10 alkyl,
   R^{23A} is (1) C1-15 alkyl, (2) C2-15 alkenyl, (3) C2-15 alkynyl or (4) C1-10 alkyl substituted by C1-10 alkoxy, C1-10 alkylthio or C1-10 alkyl-NR^{27A}-,
   R^{27A} is (1) a hydrogen atom or (2) C1-10 alkyl,
   ring1^{A}, ring2^{A}, ring5^{A}, ring6^{A} and ring7^{A} are (1) C3-15 mono-, bi- or tri-carbocyclic aryl which may be partially or fully saturated or (2) 3- to 15-membered mono-, bi- or tri-heterocyclic aryl containing 1 to 4 hetero atom(s) selected from oxygen, nitrogen and sulfur atom(s) which may be partially or fully saturated,
   ring3^{A} and ring4^{A} are (1) thienyl, (2) phenyl or (3) furyl, ring6^{A} and ring7^{A} may be substituted by 1 to 3 R^{28A},
   R^{28A} is (1) C1-10 alkyl, (2) C2-10 alkenyl, (3) C2-10 alkynyl, (4) C1-10 alkoxy, (5) C1-10 alkyl substituted by C1-10 alkoxy, (6) a halogen atom, (7) hydroxy, (8) C1-10 alkyl substituted by 1 to 3 halogen atom(s) or (9) C1-10 alkyl substituted by C1-10 alkoxy substituted by 1 to 3 halogen atom(s), and wherein (1) when T^{A} is an oxygen atom, X^{A} is CH₂-, A^{A} is A^{1A}, and D^{A} is D^{1A}, E^{A} is E^{2A}, (2) ring5^{A} is not C3-7 cycloalkyl, phenyl, thienyl nor furyl, (3) when ring6^{A} is phenyl, phenyl has at least one R^{28A},
   a salt thereof, an N-oxide thereof or a solvate thereof, or a prodrug thereof, or a cyclodextrin clathrate thereof,
8. The agent according to the above 1 or 4, wherein EP₄ agonist is a compound represented by formula (IB): 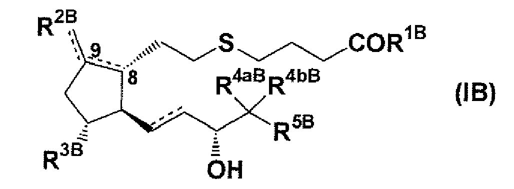 wherein R^{1B} is hydroxy, C1-6 alkyloxy or NR^{6B} R^{7B} in which R^{6B} and R^{7B} are each independently hydrogen or C1-4 alkyl,
   R^{2B} is an oxygen atom, a halogen atom or O-COR^{8B} in which R^{8B} is C1-4 alkyl, phenyl or phenyl(C1-4 alkyl),
   R^{3B} is a hydrogen atom or hydroxy,
   R^{4aB} and R^{4bB} are each independently a hydrogen atom or C1-4 alkyl, R^{5B} is phenyl substituted with the following substituent(s):
      i) 1 to 3 selected from (a) C1-4 alkyloxy-C1-4 alkyl, (b) C2-4 alkenyloxy-C1-4alkyl, (c) C2-4 alkynyloxy-C1-4 alkyl, (d) C3-7 cycloalkyloxy-C1-4alkyl, (e) C3-7 cycloalkyl(C1-4 alkyloxy)-C1-4 alkyl, (f) phenyloxy-C1-4 alkyl, (g) phenyl-C1-4 alkyloxy-C1-4 alkyl, (h) C1-4 alkylthio-C1-4 alkyl, (i) C2-4 alkenylthio-C1-4 alkyl, (j) C2-4 alkynylthio-C1-4 alkyl, (k) C3-7 cycloalkylthio-C1-4 alkyl, (I) C3-7 cycloalkyl(C1-4 alkylthio)-C1-4 alkyl, (m) phenylthio-C1-4alkyl and (n) phenyl-C1-4 alkylthio-C1-4alkyl,
      ii) (a) C1-4 alkyloxy-C1-4 alkyl and C1-4 alkyl, (b) C1-4 alkyloxy-C1-4 alkyl and C1-4 alkyloxy, (c) C1-4 alkyloxy-C1-4 alkyl and hydroxy, (d) C1-4 alkyloxy-C1-4 alkyl and halogen atom, (e) C1-4 alkylthio-C1-4 alkyl and C1-4 alkyl, (f) C1-4 alkylthio-C1-4 alkyl and C1-4 alkyloxy, (g) C1-4 alkylthio-C1-4 alkyl and hydroxy or (h) C1-4 alkylthio-C1-4 alkyl and a halogen atom,
      iii) (a) haloalkyl or (b) hydroxy-C1-4 alkyl, or iv) C1-4 alkyl and hydroxy;
         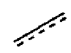 is independently a single bond or a double bond, wherein continuous double bonds are not formed, wherein when R^{2B} is O-COR^{8B}, the 8-9 position represents a double bond,
         a salt thereof, an N-oxide thereof or a solvate thereof, or a prodrug thereof, or a cyclodextrin clathrate thereof,
9. The agent according to the above 1 or 4, wherein the EP₄ agonist is the compound selected from
   ({3-[((1R,2S,3R)-3-hydroxy-2-{(1E,3S)-3-hydroxy-4-[3-(methoxymethyl)phenyl]but-1-enyl}-5-oxocyclopentyl)sulfanyl]propyl}sulfanyl)acetic acid;
   4-{[2-((1R,2R,3R)-3-hydroxy-2--{(1E,3S)-3-hydroxy-4-[3-(methoxymethyl)phenyl]but-1-enyl}-5-oxocylcopentyl)ethyl]sulfanyl}butanoic acid;
   7-((1R,2R,3R)-3-hydroxy-2-{(1E,3S)-3-hydroxy-4-[3-(methoxymethyl)phenyl]but-1-enyl}-5-oxocydopentyl)heptanoic acid;
   (5Z)-7-((1R,2R,3R)-2-{(1E,3S)-4-[3-(ethoxymethyl)phenyl]-3-hydroxybut-1-enyl}-3-hydroxy-5-oxocyclopentyl)hept-5-enoic acid;
   (5Z)-7-((1R,2R,3R,5R)-5-chloro-2-{(1E,3S)-4-[3-(ethoxymethyl)phenyl]-3-hydroxybut-1-enyl}-3-hydroxycyclopentyl)hept-5-enoic acid;
   4-[(2-{(1R,2R,3R)-3-hydroxy-2-[(1E,3S)-3-hydroxy-4-(4-hydroxy-3-methylphenyl)but-1-enyl]-5-oxocyclopentyl}ethyl)sulfanyl]butanoic acid;
   methyl 4-([2-((1R,2R,3R)-3-hydroxy-2-{(1E,3S)-3-hydroxy-4-[3-(methoxymethyl)phenyl]but-enyl}-5-oxocyclopentyl)ethyl]sulfanyl}butanoate;
   4-{[2-((1R,2R)-2-{(1E,3S)-3-hydroxy-4-[3-(methoxymethyl)phenyl]but-1-enyl}-5-oxocyclopentyl)ethyl]sulfanyl}butanoic acid;
   4-[(2-{(2R)-2-[(1E,3S)-4-(3-chlorophenyl)-3-hydroxybut-1-enyl]-5-oxopyrrolidin-1-yl}ethyl)sulfanyl]butanoic acid;
   4-{[2-((2R)-2-{(1E,3S)-3-hydroxy-4-[3-(trifluoromethyl)phenyl]but-1-enyl}-5-oxopyrrolidin-1-yl)ethyl]sulfanyl}butanoic acid;
   4-[(2-{(2R)-2-[(1E,3S)-4-(4-fluorophenyl)-3-hydroxybut-1-enyl]-5-oxopyrrolidin-1-yl}ethyl)sulfanyl]butanoic acid;
   4-[(2-{(2R)-2-[(1E,3S)-3-hydroxy-4-(2-naphthyl)-but-1-enyl]-5-oxopyrrolidin-1-yl}ethyl)sulfanyl]butanoic acid;
   4-[(2-{(4S)-4-[(1E,3S)-4-(4-fluorophenyl)-3-hydroxy-1-butenyl]-2-oxo-1,3-oxazolidin-3-yl}ethyl)sulfanyl]butanoic acid;
   2-[(2-{(2R)-2-[(1E,35)-3-hydroxy-4-(3-methylphenyl)but-1-enyl]-5-oxopyrrofidin-1-yl}ethyl)suffanyl]-1,3-thiazol-4-carboxylic acid;
   2-[(2-{(2R)-2-[(1E(3S)-3-hydroxyoct-1-enyl]-5-oxopyrrolidin-1-yl}ethyl)sulfanyl]-1,3-thiazol-4-carboxylic acid;
   2-{[2-((2R)-2-{(1E,3S)-4-[3-(1-benzofuran-2-yl)phenyl]-3-hydroxybut-1-enyl}-5-oxopyrrolidin-1-yl)ethyl]sulfanyl}-1,3-thiazol-4-carboxylic acid;
   4-[(2-{(2R)-2-[(1E,35)-3-hydroxyocat-1-enyl]-5-oxopyrrolidin-1-yl}ethyl)sulfanyl]butanoic acid;
   {[3-({(1R,25,3R)-3-hydroxy-2-[(1E,3S)-3-hydroxyoct-1-enyl]-5-oxocyclopentyl}sulfanyl)propyl]sulfanyl}acetic acid; and
   2-[(2-{(4S)-4-[(1E,3S)-4-(4-fluorophenyl)-3-hydroxybut-1-enyl]-2-oxo-1,3-oxazolidin-3-yl}ethyl)sulfanyl]-1,3-thiazol-4-carboxylic acid,
   a salt thereof, an N-oxide thereof or a solvate thereof, or a prodrug thereof, or a cyclodextrin clathrate thereof,
10. A method for preventing and/or treating for hyperkalemia which comprises administering an effective amount of EP₄ agonist to a mammal,
11. A method for promotion of potassium excretion which comprises administering an effective amount of EP₄ agonist to a mammal,
12. Use of EP₄ agonist for preparing a preventive and/or therapeutic agent for hyperkalemia, and
13. Use of EP₄ agonist for preparing a potassium excretion promoter.

In the specification, the "cyclic ring" in the "cyclic ring group which may have a substituent(s)" represented by R⁴ includes, for example, carbocyclic ring or heterocyclic ring. The carbocyclic ring includes, for example, C3-15 mono- or poly-carbocyclic ring, spiro-linked poly-carbocyclic ring or bridged poly-carbocyclic ring *etc*. C3-15 mono- or poly-carbocyclic ring includes C3-15 mono- or poly-carbocyclic ring which is unsaturated or saturated partially or fully thereof.

For example, cyclopropane, cyclobutane, cyclopentane, cyclohexane, cycloheptane, cyclooctane, cyclononane, cyclodecane, cycloundecane, cyclododecane, cyclotridecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indan, naphthalene, dihydronaphthalene, teterahydronaphthalene, perhydronaphthalene, heptalene, perhydroheptalene, biphenylene, asindacene, s-indacene, acenaphthylene, acenaphthene, fluorene, phenalene, phenanthrene, anthracene ring *etc*. are included.

Spiro-linked poly-carbocyclic ring includes, for example, spiro[4.4]nonane, spiro[4,5]decane, spiro[5.5]undecane ring *etc*. Bridged polycarbocyclic ring includes, for example, bicyclo[2.2.1]heptane, bicyclo[2.2.1]hept-2-ene, bicyclo[3.1.1]heptane, bicyclo[3.1.1]hept-2-ene, bicyclo[3.2.1]octane, bicydo[2.2.2]octane, bicyclo[2.2.2]oct-2-ene, adamantane, noradamantane ring *etc*. Among them, C3-15 mono- or polyaromatic carbocyclic ring includes, for example, benzene, azulene, naphthalene, phenanthrene, anthracene ring *etc*.

Heterocyclic ring includes, for example, 3 to 15 membered mono- or poly-heterocyclic ring, spiro-linked poly-heterocyclic ring or bridged poly-heterocyclic ring. These rings comprise 1 to 5 hetero atom(s) selected from oxygen, nitrogen and/or sulfur. 3 to 15 membered mono- or poly-heterocyclic ring which comprises 1 to 5 hetero atom(s) selected from oxygen, nitrogen and/or sulfur includes 3 to 15 membered mono- or poly-heterocyclic ring comprising 1 to 5 hetero atom(s) selected from oxygen, nitrogen and/or sulfur, which is unsaturated or saturated partially or fully thereof.

For example, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzoxepine, benzoxazepine, benzoxadiazepine, benzothiepine, benzothiazepine, benzothiadiazepine, benzazepine, benzodiazepine, benzofurazan, benzothiadiazole, benzotriazole, carbazole, β-carboline, acridine, phenazine, dibenzofuran, xanthene, dibenzothiophene, phenothiazine, phenoxazine, phenoxathiin, thianthrene, phenanthridine, phenanthroline, perimidine, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, dioxaindan, benzodioxane, chroman, benzodithiolane, benzodithiane ring *etc*. are included.

Spiro-linked bi-heterocyclic ring includes, for example, azaspiro[4,4]nonane, azaspiro[4.5]decane, azaspiro[5.5]undecane ring *etc*. Bridged biheterocyclic ring includes, for example, azabicyclo[2.2.1.]heptane, azabicydo[3.I. I]heptane, azabicyclo[3.2.1]octane, azabicyclo[2.2.2]octane ring *etc*.

Among them, 3 to 15 membered mono-, bi- or tri-aromatic heterocyclic ring which comprises 1 to 5 hetero atom(s) selected from oxygen, nitrogen and/or sulfur includes for example pyrrole, imidazole, triazole, tetrazole, pyrazole, pyridine, pyrazine, pyrimidine, pyridazine, furan, thiophene, oxazole, isoxazole, thiazole, isothiazole, furazan, oxadiazole, thiadiazole, indole, isoindole, benzofuran, isobenzofuran, benzothiophene, isobenzothiophene, indazole, quinoline, isoquinoline, purine, phthalazine, pteridine, naphthyridine, quinoxaline, quinazoline, cinnoline, benzoxazole, benzothiazole, benzimidazole, benzofurazan, benzothiadiazole, benzotriazole, carbazole, β-carboline, acridine, phenazine, dibenzofuran, dibenzothiophene, phenanthridine, phenanthroline, perimidine ring *etc*.

The "substituent" in the "cyclic ring group which may have a substituent(s)" represented by R⁴ includes, for example, (a) alkyl group which may have a substituent(s), (b) alkenyl group which a may have substituent(s), (c) alkynyl group which may have a substituent(s), (d) carbocyclic ring which may have a substituent(s), (e) heterocyclic ring may have a substituent(s), (f) hydroxy group may have a substituent(s), (g) thiol group which may have a substituent(s), (h) amino group which may have a substituent(s), (i) carbamoyl group which may have a substituent(s), (j) sulfamoyl group which may have a substituent(s), (k) carboxy group, (I) alkoxycarbonyl group (for example, C1-6 alkoxycarbonyl such as methoxycarbonyl, ethoxycarbonyl, t-butoxycarbonyl *etc*.), (m) sulfo group (-SO₃H), (n) sulfino group (-SO₂H), (o) phosphono group (-PO(OH)₂), (p) nitro group, (q) oxo group, (r) thioxo group, (s) cyano group, (t) amidino group, (u) imino group, (v) -B(OH)₂ group, (w) halogen group, (for example, fluorine, chlorine, bromine, iodine *etc*.), (x) alkylsulfinyl group (for example, C1-6 alkylsulfinyl group such as methylsulfinyl, ethylsulfinyl *etc.*), (y) arylsulfinyl group (for example, C6-10 arylsulfinyl group such as phenylsulfinyl *etc*.), (z) alkylsulfonyl group (for example, C1-6 alkylsulfonyl group such as methylsulfonyl, ethylsulfonyl *etc*.), (aa) arylsulfonyl (for example, C6-10 arylsulfonyl such as phenylsulfonyl *etc*.), (bb) acyl group (for example, C1-10 alkanoyl such as formyl, acetyl, propanoyl, pivaroyl *etc.,* C6-10 arylcarbonyl such as benzoyl *etc*.) *etc*. These optional substituents may be substituted 1-5 at the replaceable position.

The "alkyl group" in "alkyl group which may have a substituent(s)" as the substituent includes, for example, straight or branched C1-15 alkyl group such as methyl, ethyl, propyl, isopropyl, butyl, isobutyl, sec-butyl, tert-butyl, pentyl, isopentyl, neopentyl, hexyl, heptyl, octyl, nonyl, decyl, undecyl, dodecyl, tridecyl, tetradecyl, pentadecyl group *etc*.

The substituent of alkyl group includes hydroxy group, amino group, carboxy group, cyano group, nitro group, mono- or di-C1-10 alkylamino group (for example, methylamino, ethylamino, propylamino, dimetylamino, diethylamino *etc*.), C1-10 alkoxy group (for example, methoxy, ethoxy, propoxy, isopropoxy, t-butoxy, hexyloxy, octyloxy, decanyloxy *etc*), C1-6 acyloxy group (for example, acetyloxy, ethanoyloxy, propanoyloxy, butanoyloxy, pentanoyloxy, hexanoyloxy group *etc*.), C1-10 alkylcarbonyloxy group (for example, acetoxy, ethylcarbonyloxy *etc*.), carbocyclic ring (it has the same meaning as the above-mentioned carbocyclic ring in the "cyclic ring" of the "cyclic ring group which may have a substituent(s)".), heterocyclic ring (it has the same meaning as the above-mentioned heterocyclic ring in the "cyclic ring" of the "cyclic ring group which may have a substituent(s)".), halogen atom (it has the same meaning as mentioned above.), C1-10 alkoxy substituted by 1 to 3 halogen atom(s) (for example, monofluoromethoxy group, difluoromethoxy group, trifluoromethoxy group *etc*.), -O-carbocyclic ring (it has the same meaning as the above-mentioned carbocyclic ring in the "cyclic ring" of the "cyclic ring group which may have a substituent(s)".), and -O-heterocylic ring (it has the same meaning as the above-mentioned heterocyclic ring in the "cyclic ring" of the "cyclic ring group which may have a substituent(s)".) *etc*. These optional substituents may be substituted 1-4 at the replaceable position.

The "alkenyl group" in "alkenyl group which may have a substituent(s)" as the substituent includes, for example, straight or branched C2-15 alkenyl group such as ethenyl, propenyl, butenyl, butadienyl, pentenyl, pentadienyl, hexenyl, hexadienyl, heptenyl, heptadienyl, octenyl, octadienyl, nonenyl, nonadienyl, decenyl, decadienyl, undecenyl, dodecenyl, tridecenyl, tetradecenyl, pentadecenyl *etc*. The substituent of alkenyl group has the same meaning as the above-mentioned the substituent in "alkyl group which may have a substituent(s)".

The "alkynyl group" in "alkynyl group which may have a substituent(s)" as the substituent includes, for example, straight or branched C2-15 alkynyl group such as ethynyl, propynyl, butynyl, butadiynyl, pentynyl, pentadiynyl, hexynyl, hexadiynyl, heptynyl, heptadiynyl, octynyl, octadiynyl, nonynyl, nonadiynyl, decynyl, decadiynyl, undecynyl, dodecynyl, tridecynyl, tetradecynyl, pentadecynyl *etc*. The substituent of alkynyl group has the same meaning as the above-mentioned the substituent in "alkyl group which may have a substituent(s)".

The "carbocyclic ring" in "carbocyclic ring which may have a substituent(s)" as the substituent has the same meaning as the above-mentioned carbocyclic ring in the "cyclic ring" of the "cyclic ring group which may have a substituent(s)". The substituent of carbocyclic ring includes, for example, straight or branched C1-15 alkyl group (it has the same meaning as the above-mentioned alkyl group of the "alkyl group which may have a substituent(s)".), straight or branched C2-15 alkenyl group (it has the same meaning as the above-mentioned alkenyl group of the "alkenyl group which may have a substituent(s)".), straight or branched C2-15 alkynyl group (it has the same meaning as the above-mentioned alkynyl group of the "alkynyl group which may have a substituent(s)".), hydroxy group, C1-6 alkoxy group (for example, methoxy, ethoxy, propoxy, isopropoxy, butoxy, isobutyloxy, tert-butoxy, pentyloxy, hexyloxy *etc*.), thiol group, C1-6 alkylthio group (for example, methylthio, ethylthio, propylthio, isopropylthio, butylthio, isobutylthio, isobutylthio, tert-butylthio, pentylthio, hexylthio *etc*.), amino group, mono- or di-C1-6 alkylamino group (for example, methylamino, ethylamino, propylamino, isopropylamino, butylamino, isobutylamino, tert-butylamino, pentylamino, hexylamino, dimethylamino, diethylamino, dipropylamino, N-methyl-N-ethylamino *etc*), halogen atom (it has the same meaning as mentioned above.), cyano group, nitro group, trifluoromethyl group, trifluoromethoxy group *etc*. These optional substituents may be substituted 1-5 at the replaceable position.

The "heterocyclic ring" in "heterocyclic ring which may have a substituent(s)" as the substituent has the same meaning as the above-mentioned heterocyclic ring in the "cyclic ring" of the "cyclic ring group which may have a substituent(s)".

The "substituent" in "hydroxy group which may have a substituent(s)", "thiol group which may have a substituent(s)" and "amino group which may have a substituent(s)" as the substituent includes, for example, (i) alkyl group which may have a substituent(s) (it has the same meaning as mentioned above.), (ii) alkenyl group which may have a substituent(s) (it has the same meaning as mentioned above.), (iii) alkynyl group which may have a substituent(s) (it has the same meaning as mentioned above.), (iv) carbocyclic ring which may have a substituent(s) (it has the same meaning as mentioned above.), (v) heterocyclic ring which may have a substituent(s) (it has the same meaning as mentioned above.), (vi) acyl group (for example, C1-6 alkanoyl group such as formyl, acetyl, propanoyl, pivaloyl, butanoyl, pentanoyl, hexanoyl *etc*. or isomer thereof, or C6-10 aromatic carbocyclic ring carbonyl such as benzoyl *etc*.), (vii) carbamoyl group which may have a substituent(s) (it has the same meaning as mentioned below.), (viii) alkylsulfonyl group (for example, C1-6 alkyl sulfonyl group such as methylsulfonyl, ethylsulfonyl *etc*.), (ix) arylsulfonyl group (for example, C6-10 arylsulfonyl such as phenylsulfonyl *etc*.).

The "carbamoyl group which may have a substituent(s)" as the substituent includes carbamoyl group which have no substituent, N-mono-C1-6 alkylcarbamoyl (for example, N-methylcarbamyl, N-ethylcarbamyl, N-propylcarbamoyl, N-isopropylcarbamoyl, N-butylcarbamoyl, N-isobutylcarbamoyl, N-(tert-butyl)carbamoyl, N-pentylcarbamoyl, N-hexylcarbamoyl *etc*.), N-mono-C6-10 arylcarbamyl such as N-phenylcarbamoyl *etc*., N,N-di-C1-6 alkylcarbamoyl (for example, N,N-dimethylcarbamoyl, N,N-diethylcarbamoyl, N,N-dipropylcarbamoyl, N,N-dibutylcarbamoyl, N,N-dipentylcarbamoyl, N,N-dihexylcarbamoyl, N-methyl-N-ethylcarbamoyl *etc*.), N-di-C6-10 arylcarbamoyl such as N,N-diphenylcarbamoyl *etc*., N-C6-10 aryl-N-C1-6 alkylcarbamoyl (for example, N-phenyl-N-methylcarbamoyl, N-phenyl-N-ethylcarbamoyl, N-phenyl-N-propylcarbamoyl, N-phenyl-N-butylcarbamoyl, N-phenyl-N-pentylcarbamoyl, N-phenyl-N-hexylcarbamoyl etc*.*).

The "sulfamoyl group which may have a substituent(s)" as the substituent includes sulfamoyl group which have no substituent, N-mono-C1-6 alkylsulfamoyl (for example, N-methylsulfamoyl, N-ethylsulfamoyl, N-propylsulfamoyl, N-isopropylsulfamoyl, N-butylsulfamoyl, N-isobutylsulfamoyl, N-(tert-butyl)sulfamoyl, N-pentylsulfamoyl, N-hexylsulfamoyl *etc*.), N-mono-C6-10 arylsulfamoyl such as N-phenylsulfamoyl *etc*., N,N-di-C1-6 alkylsulfamoyl (for example, N,N-dimethylsulfamoyl, N,N-diethylsulfamoyl, N,N-dipropylsulfamoyl, N,N-dibutylsulfamoyl, N,N-dipentylsulfamoyl, N,N-dihexylsulfamoyl, N-methyl-N-ethylsulfamoyl *etc*.), N-di-C6-10 arylsulfamoyl such as N,N-diphenylsulfamoyl *etc*., N-C6-10 aryl-N-C1-6 alkylsulfamoyl (for example, N-phenyl-N-methylsulfamoyl, N-phenyl-N-ethylsulfamoyl, N-phenyl-N-propylsulfamoyl, N-phenyl-N-butylsulfamoyl, N-phenyl-N-pentylsulfamoyl, N-phenyl-N-hexylsulfamoyl *etc.).*

In the specification, the "aliphatic hydrocarbon group which may have a substituent(s)" represented by R⁴ includes, for example, alkyl group which may have a substituent(s), alkenyl group which may have a substituent(s) or alkynyl group which may have a substituent(s). The "alkyl group which may have a substituent(s)", the "alkenyl group which may have a substituent(s)" or the "alkynyl group which may have a substituent(s)" has the same meaning as the above-mentioned "alkyl group which may have a substituent(s)", "alkenyl group which may have a substituent(s)" or "alkynyl group which may have a substituent(s)" defined in substituent of cyclic ring.

In the specification, the "C1-6 alkyloxy group" represented by R¹ includes, for example, methoxy, ethoxy, propoxy, butoxy, pentyloxy, hexyloxy *etc*.

In the specification, the "C1-6 acyloxy group" represented by R¹ includes, for example, acetyloxy, ethanoyloxy, propanoyloxy, butanoyloxy, pentanoyloxy, hexanoyloxy *etc*.

In the specification, the "alkyl group which may have a substituent(s)" represented by R² or R³ has the same meaning as the above-mentioned "alkyl group which may have a substituent(s)" defined in R⁴ .

In the specification, the "halogen atom" represented by R₂ or R³ has the same meaning as mentioned above.

In the specification, the "halogen atom" represented by T has the same meaning as mentioned above.

In the specification, the "acyloxy group" in "acyloxy group which may have a substituent(s)" represented by T includes, for example, C1-10 alkanoyloxy group such as acetyloxy, propanoyloxy, pivaloyloxy *etc*. The "substituent" in "acyloxy group which may have a substituent(s)" represented by T has the same meaning as the above-mentioned "alkyl group which may have a substituent(s)" defined in R⁴.

In the specification, the "substituent" in "nitrogen atom which may have a substituent(s)" represented by X and Y includes "alkyl group which may have a substituent(s)" or "cyclic ring group which may have a substituent(s)". The "alkyl group which may have a substituent(s)" or the "cyclic ring which may have a substituent(s)" has the same meaning as the above-mentioned "alkyl group which may have a substituent(s)" or "cyclic ring group which may have a substituent(s)" defined in R⁴. In the specification, the "spacer of which main chain has an atom number of 1-8 " in "spacer of which main chain has an atom number of 1-8 and which may have a substituent(s)" represented by A means the distance that 1-8 atom(s) of main chain is(are) connected. In this case, the "atom number of main chain" should be counted atom number of main chain to become minimal. For example, the atom number is counted as 4 or 3 in 1,4-phenylene or 1,3-phenylene respectively.

The "spacer of which main chain has an atom number of 1-8" includes, for example, C1-8 alkylene, C2-8 alkenylene, C2-8 alkynylene, cyclic ring *etc*. The carbon atom in these groups may be replaced with 1 to 5 oxygen atom, nitrogen atom, sulfur atom, carbonyl group, thiocarbonyl group, sulfinyl group or sulfonyl group at replaceable positions structurally. C1-8 alkylene group includes, for example, methylene, ethylene, trimethylene, tetramethylene, pentamethylene, hexamethylene, heptamethylene, octamethylene *etc*. C2-8 alkenylene includes, for example, ethenylene, propenylene, butenylene, butadienylene, pentenylene, pentadienylene, hexenylene, hexadienylene, heptenylene, heptadienylene, octenylene, octadienylene *etc*. C2-8 alkynylene group includes, for example, ethynylene, propynylene, butynylene, butadiynylene, pentynylene, pentadiynylene, hexynylene, hexadiynylene, heptynylene, heptadiynylene, octynylene, octadiynylene *etc*.

The cyclic ring has the same meaning as the above-mentioned "cyclic ring" in "cyclic ring group which may have a substituent(s)" defined in R⁴. The "substituent" in "spacer of which main chain has an atom number of 1-8 and which may have a substituent(s)" has the same meaning as the above-mentioned "substituent" in "alkyl group which may have a substituent(s)". These optional substituents may be substituted 1-10, preferably 1-5, more preferably 1-3 at the replaceable position.

In the specification, the "acidic group which may be protected" represented by D represents the "acidic group" which may be protected by a "protecting group". Examples of the "acidic group" include (a) hydroxy group, (b) formyl group (-CHO), (c) alkoxy group (for example, C1-6 alkoxy group such as methoxy, ethoxy, propoxy, isopropoxy, butoxy, isobutyloxy, tert-butoxy, pentyloxy, hexyloxy *etc*., C6-10 aryloxy such as phenoxy), (d) carboxy (-COOH), (e) sulfo (-SO₃H), (f) sulfonamide (-SO₂NH₂ or - NR¹⁰¹SO₃H (R¹⁰¹ is hydrogen atom or alkyl group which may have a substituent(s). (it has the same meaning as the above-mentioned "alkyl group which may have a substituent(s)" defined in R⁴.)), (g) phosphono (-PO(OH)₂), (h) phenol (-C₆H₄OH), (i) -CONR¹⁰¹SO₃H (R¹⁰¹ has the same meaning as mentioned above.), (j) amido (-CONH₂) or (k) various types of Brønsted acid such as a nitrogen-containing ring residue having hydrogen from which can be removed as proton.

The "Brønsted acid" means a substance which gives hydrogen ion to other substance. Examples of the "nitrogen-containing ring residue having hydrogen from which can be removed as proton" include: 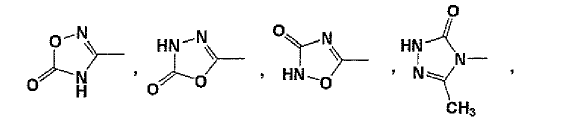 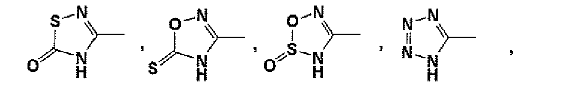 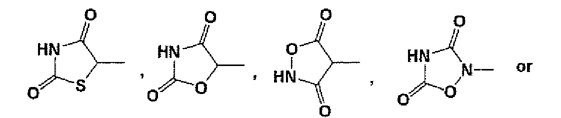 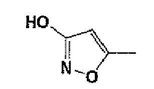 *etc*. Preferred as "acidic group" is carboxy group or hydroxy group. More preferred is carboxy group.

The "protecting group" in "acidic group which may be protected" represented by D includes (a) alkyl group which may have a substituent(s) (it has the same meaning as the above-mentioned "alkyl group which may have a substituent(s)" defined in R⁴), (b) cyclic ring group which may have a substituent(s) (it has the same meaning as the above-mentioned "cyclic ring group which may have a substituent(s)" defined in R⁴), (c) amino group which may have a substituent(s) (it has the same meaning as the above-mentioned "amino group which may have a substituent(s)" defined in R⁴), (d) amino acid, (e) acyl group which may have a substituent(s) *etc*.

The "acyl group" in "acyl group which may have a substituent(s)" has the same meaning as the above-mentioned "acyl group" defined in R⁴. The "substituent" in "acyl group which may have a substituent(s)" has the same meaning as the above-mentioned substituent in "alkyl group which may have a substituent(s)" defined in R⁴.

The "amino acid" means a natural amino acid or unnatural amino acid residue. The natural amino acid or unusual amino acid residue includes, for example, glycine, alanine, valine, leucine, isoleucine, serine, threonine, cystein, methionine, proline, asparagine, glutamine, phenylalanine, tyrosine, tryptophan, aspartic acid, glutamic acid, lysine, arginine, histidine, β-alanine, cystathionine, cystine, homoserine, isoleucine, lanthionine, norleucine, norvaline, ornithine, sarcosine, thyronine *etc*. When the amino acid contains an amino group, the amino acid which is substituted by the above-mentioned substituent of amino group is included.

EP₄ agonists in the present invention do not only include ones which have ever been found but ones which will be found from now. EP₄ agonists which have ever been found include, for example, the compounds described in the following (A)-(W).
(A) In the pamphlet of WO03/009872, it is described that the compound represented by the following formula (IA) has EP₄ agonistic activity. As well, the definition of each group of the compound represented by formula (IA) is described in the pamphlet of WO03/009872 in detail. Accordingly, EP₄ agonist of the present invention includes the compound represented by formula (IA) : 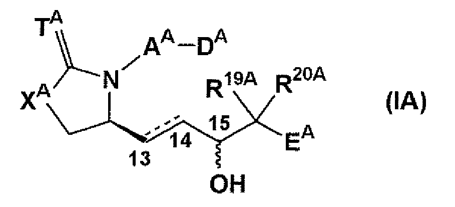 wherein 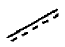 is (1) a single bond or (2) a double bond,
   R^{19A} and R^{20A} are each independently, (1) a hydrogen atom, (2) C1-10 alkyl or (3) a halogen atom,
   T^{A} is (1) an oxygen atom or (2) a sulfur atom,
   X ^{A} is (1) -CH₂-, (2) -O- or (3) -S-,
   A^{A} is A^{1A} or A^{2A}, A^{1A} is (1) C2-8 straight-chain alkylene which may be substituted by 1 to 2 C1-4 alkyl, (2) C2-8 straight-chain alkenylene which may be substituted by 1 to 2 C1-4 alkyl or (3) C2-8 straight-chain alkynylene which may be substituted by 1 to 2 C1-4 alkyl,
   A^{2A} is -G^{1A} -G^{2A} -G^{3A}
   G^{1A} is (1) C1-4 straight-chain alkylene which may be substituted by 1 to 2 C1-4 alkyl, (2) C2-4 straight-chain alkenylene which may be substituted by 1 to 2 C1-4 alkyl or (3) C2-4 straight-chain alkynylene which may be substituted by 1 to 2 C1-4 alkyl,
   G^{2A} is (1) -Y^{A} -, (2) -(ring1^{A})-, (3) -Y^{A}-(ring1⁴)-, (4) -(ring1^{A})-Y^{A}- or (5) -Y^{A}-(C1-4 alkylene)-(ring1^{A})-, Y^{A} is (1) -S-, (2) -SO-, (3) -SO₂-, (4) -O-or (5) -NR^{1A}-,
   R^{1A} is (1) a hydrogen atom, (2) C1-10 alkyl or (3) C2-10 acyl,
   G^{3A} is (1) a bond, (2) C1-4 straight-chain alkylene which may be substituted by 1 to 2 C1-4 alkyl, (3) C2-4 straight-chain alkenylene which may be substituted by 1 to 2 C1-4 alkyl or (4) C2-4 straight-chain alkynylene which may be substituted by 1 to 2 C1-4 alkyl,
   D^{A} is D^{1A} or D^{2A},
   D^{1A} is (1) -COOH, (2) -COOR^{2A}, (3) tetrazol-5-yl or (4) CONR^{3A}SO₂R^{4A},
   R^{2A} is (1) C1-10 alkyl, (2) phenyl, (3) C1-10 alkyl substituted by phenyl or (4) biphenyl,
   R^{3A} is (1) a hydrogen atom or (2) C1-10 alkyl,
   R^{4A} is (1) C1-10 alkyl or (2) phenyl,
   D^{2A} is (1) -CH₂OH, (2) -CH₂OR^{5A}, (3) hydroxy, (4) -OR^{5A}, (5) formyl, (6) -CONR^{6A}R^{7A}, (7) -CONR^{6A}SO₂R^{8A}, (8) -CO-(NH-amino acid residue-CO)_{mA} -OH, (9) -O-(CO- amino acid residue -NH)_{mA} -H, (10) -COOR^{9A}, (11) -OCO-R^{10A}, (12) -COO-Z^{1A} -Z^{2A} -Z^{3A}, (13) 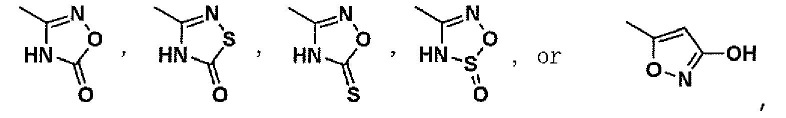
   R^{5A} is C1-10 alkyl,
   R^{6A} and R^{7A} are each independently, (1) a hydrogen atom or (2) C1-10 alkyl,
   R^{8A} is C1-10 alkyl substituted by phenyl,
   R^{9A} is (1) C1-10 alkyl substituted by biphenyl which may be substituted by 1 to 3 C1-10 alkyl, C1-10 alkoxy or halogen atom or (2) biphenyl substituted by 1 to 3 C1-10 alkyl, C1-10 alkoxy or halogen atom,
   R^{10A} is (1) phenyl or (2) C1-10 alkyl, mA is 1 or 2,
   Z^{1A} is (1) C1-15 alkylene, (2) C2-15 alkenylene or (3) C2-15 alkynylene,
   Z^{2A} is (1) -CO-, (2) -OCO-, (3) -COO-, (4) -CONR^{11A}-, (5) -NR^{12A}CO-, (6) -O-, (7) -S-, (8) -SO-, (9) -SO₂ (10) -NR^{13A}-, (11) -NR^{14A}CONR^{15A}-, (12) -NR^{16A}COO-, (13) -OCONR^{17A}- or (14) -OCOO-,
   Z^{3A} is (1) a hydrogen atom, (2) C1-15 alkyl, (3) C2-15 alkenyl, (4) C2-15 alkynyl, (5) ring^{2A} or (6) C1-10 alkyl substituted by C1-10 alkoxy, C1-10 alkylthio, C1-10 alkyl-NR^{18A}- or ring2^{A},
   R^{11A}, R^{12A}, R^{13A}, R^{14A}, R^{15A}, R^{16A}, R^{17A} and R^{18A} are each independently (1) a hydrogen atom or (2) C1-15 alkyl,
   R^{11A} and Z^{3A} may be taken together with the nitrogen atom to which they are attached to form 5- to 7-membered saturated mono-heterocyclic ring, and the heterocyclic ring may contain other one hetero atom selected from oxygen, nitrogen and sulfur atom(s),
   E^{A} is E^{1A} or E^{2A} ,
   E^{1A} is (1) C3-7 cycloalkyl or (2) ring3^{A},
   E^{2A} is (1) C3-7 cycloalkyl, (2) ring4^{A} or (3) ring5^{A},
   ring1^{A} and ring5^{A} which may be substituted by 1 to 3 R^{21A} and/or R^{22A} ,
   ring3^{A} may be substituted by 1 to 2 R^{21A},
   C3-7 cycloalkyl represented by E^{2A} is substituted by one of R^{21A} or R^{22A,} and may be substituted by another 1 to 2 R^{21A} and/or R^{22A},
   ring4^{A} is substituted by one of R^{22A}, may be substituted by another 1 to 2 R^{21A} and/or R^{22A}, and may be substituted by heterocyclic ring formed by R¹¹A
   Z^{3A} and the nitrogen to which Z^{3A} is attached or ring2^{A} may be substituted by R^{23A},
   R^{21A} is (1) C1-10 alkyl, (2) C1-l0 alkoxy, (3) a halogen atom, (4) nitro, (5) C1-10 alkyl substituted by 1 to 3 halogen atom(s) or (6) phenyl,
   R^{22A} is (1) C2-10 alkenyl, (2) C2-10 alkynyl, (3) C1-10 alkylthio, (4) hydroxy, (5) -NR^{24A}R^{25A}, (6) C1-10 alkyl substituted by C1-10 alkoxy, (7) C1-10 alkyl substituted by C1-10 alkoxy substituted by 1 to 3 halogen atom(s), (8) C1-10 alkyl substituted by -NR^{24A}R^{25A}, (9) ring6^{A}, (10) -O-ring7^{A}, (11) C1-10 alkyl substituted by ring7^{A}, (12) C2-10 alkenyl substituted by ring7 ^{A}, (13) C2-10 alkynyl substituted by ring7^{A}, (14) C1-10 alkoxy substituted by ring7^{A}, (15) C1-10 alkyl substituted by -O-ring7^{A}, (16) -COOR ^{26A} or (17) C1-10 alkoxy substituted by 1 to 3 halogen atom(s),
   R^{24A}, R^{25A} and R^{26A} are each independently, (1) a hydrogen atom or (2) C1-10 alkyl,
   R^{23A} is (1) C1-15 alkyl, (2) C2-15 alkenyl, (3) C2-15 alkynyl or (4) C1-10 alkyl substituted by C1-10 alkoxy, C1-10 alkylthio or C1-10 alkyl-NR^{27A}- ,
   R^{27A} is (1) a hydrogen atom or (2) C1-10 alkyl,
   ring1^{A}, ring2^{A}, ring5^{A}, ring6^{A} and ring7^{A} are (1) C3-15 mono-, bi- or tri-carbocyclic aryl which may be partially or fully saturated or (2) 3- to 15-membered mono-, bi- or tri-heterocyclic aryl containing 1 to 4 hetero atom(s) selected from oxygen, nitrogen and sulfur atom(s) which may be partially or fully saturated,
   ring3^{A} and ring4^{A} are (1) thienyl, (2) phenyl or (3) furyl,
   ring6^{A} and ring7^{A} may be substituted by 1 to 3 R^{28A},
   R^{28A} is (1) C1-10 alkyl, (2) C2-10 alkenyl, (3) C2-10 alkynyl, (4) C1-10 alkoxy, (5) C1-10 alkyl substituted by C1-10 alkoxy, (6) halogen atom, (7) hydroxy, (8) C1-10 alkyl substituted by 1 to 3 halogen atom(s) or (9) C1-10 alkyl substituted by C1-10 alkoxy substituted by 1 to 3 halogen atom(s), and wherein (1) when T^{A} is an oxygen atom, X^{A} is CH₂-, A^{A} is A^{1A} and D^{A} is D^{1A}, E^{A} is E^{2A}, (2) ring5^{A} is not C3-7 cycloalkyl, phenyl, thienyl nor furyl, (3) when ring6^{A} is phenyl, the phenyl has at least one R^{28A},
   a salt thereof, an N-oxide thereof or a solvate thereof, or a prodrug thereof, or a cyclodextrin clathrate thereof.
(B) In the pamphlet of WO00/003980, it is described that the compound represented by the following formula (IB) has EP₄ agonistic activity. As well, the definition of each group of the compound represented by formula (IB) is described in the pamphlet of WO00/003980 in detail. Accordingly, EP₄ agonist of the present invention includes the compound represented by formula (IB): 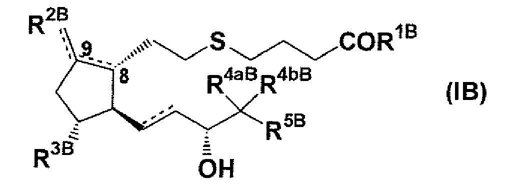 wherein R^{1B} is hydroxy, C1-6 alkyloxy or NR^{6B} R^{7B} in which R^{6B} and R^{7B} are each independently hydrogen or C1-4 alkyl,
   R^{2B} is an oxygen atom, a halogen atom or O-COR^{8B} in which R^{8B} is C1-4 alkyl, phenyl or phenyl(C1-4 alkyl),
   R^{3B} is a hydrogen atom or hydroxy,
   R^{4aB} and R^{4bB} are each independently a hydrogen atom or C1-4 alkyl,
   R^{5B} is phenyl substituted with the following substituent(s):
      i) 1 to 3 selected from (a) C1-4 alkyloxy-C1-4 alkyl, (b) C2-4 alkenyloxy-C1-4 alkyl, (c) C2-4 alkynyloxy-C1-4 alkyl, (d) C3-7 cycloalkyloxy-C1-4 alkyl, (e) C3-7 cycloalkyl(C1-4 alkyloxy)-C1-4 alkyl, (f) phenyloxy-C1-4 alkyl, (g) phenyl-C1-4 alkyloxy-C1-4 alkyl, (h) C1-4 alkylthio-C1-4 alkyl, (i) C2-4 alkenylthio-C1-4 alkyl, (j) C2-4 alkynylthio-C1-4 alkyl, (k) C3-7 cycloalkylthio-C1-4 alkyl, (1) C3-7 cycloalkyl(C1-4 alkylthio)-C1-4 alkyl, (m) phenylthio-C1-4alkyl and (n) phenyl-C1-4 alkylthio-C1-4 alkyl,
      ii) (a) C1-4 alkyloxy-C1-4 alkyl and C1-4 alkyl, (b) C1-4 alkyloxy-C1-4 alkyl and C1-4 alkyloxy, (c) C1-4 alkyloxy-C1-4 alkyl and hydroxy, (d) C1-4 alkyloxy-C1-4 alkyl and halogen atom, (e) C1-4 alkylthio-C1-4 alkyl and C1-4 alkyl, (f) C1-4 alkylthio-C1-4 alkyl and C1-4 alkyloxy, (g) C1-4 alkylthio-C1-4 alkyl and hydroxy or (h) C1-4 alkylthio-C1-4 alkyl and a halogen atom,
      iii) (a) haloalkyl or (b) hydroxy-C1-4 alkyl, or iv) C1-4 alkyl and hydroxy;
         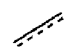 is independently a single bond or a double bond, and wherein continuous double bonds are not formed wherein when R^{2B} is O-COR^{8B}, the 8-9 position represents a double bond,
      a salt thereof, an N-oxide thereof or a solvate thereof, or a prodrug thereof, or a cyclodextrin clathrate thereof.
(C) In the specification of EP855389, it is described that the compound represented by the following formula (IC) has EP₄ agonistic activity. As well, the definition of each group of the compound represented by formula (IC) is described in the specification of EP855389 in detail. Accordingly, EP₄ agonist of the present invention includes the compound represented by formula (IC): 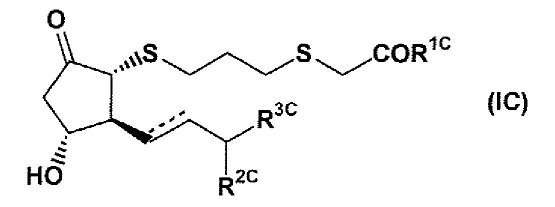 wherein R^{1C} is hydroxy, C1-4 alkoxy or NR^{6C}R^{7C} wherein R^{6C} and R^{7C} are each independently hydrogen atom or C1-4 alkyl,
   R^{2C} is a hydrogen atom or hydroxy,
   R^{3C} is (i) C1-8 alkyl, C2-8 alkenyl, or C2-8 alkynyl, (ii) phenyl or C3-7 cycloalkyl, (iii) C1-8 alkyl, C2-8 alkenyl, or C2-8 alkynyl substituted by phenyl or C3-7 cycloalkyl with the proviso that alkyl, alkenyl or alkynyl in (i) and (iii) may be substituted one hydroxy, when R^{2C} is a hydrogen atom,
      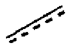 is a double bond or a single bond wherein the formula including the 8-epi equilibrium compound thereof,
   a salt thereof, an N-oxide thereof or a solvate thereof, or a prodrug thereof, or a cyclodextrin clathrate thereof.
(D) In the specification of EP985663, it is described that the compound represented by the following formula (ID) has EP₄ agonistic activity. As well, the definition of each group of the compound represented by formula (ID) is described in the specification of EP985663 in detail. Accordingly, EP₄ agonist of the present invention includes the compound represented by formula (ID): 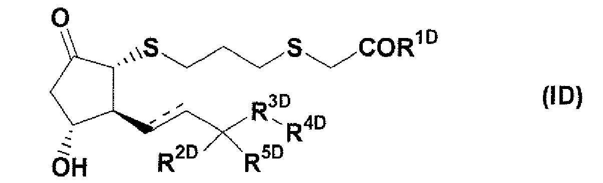 wherein R^{1D} is hydroxy, C1-6 alkyloxy or NR^{6D}R^{7D} wherein R^{6D} and R^{7D} are each independently a hydrogen atom or C1-6 alkyl;
   R^{2D} is a hydrogen atom or hydroxy;
   R^{3D} is a bond or C1-6 alkylene;
   R^{4D} is
      (i) C1-8 alkyl, C2-8 alkenyl or C2-8 alkynyl substituted by 1 to 3 substituents selected from C1-6 alkyloxy and halogen atom(s),
      (ii) phenyloxy or C3 -7 cycloalkyloxy,
      (iii) furyl, furyloxy, thienyl, thienyloxy, naphthyl, naphthyloxy, phthalanyl or phthalanyloxy,
      (iv) phenyl, phenyloxy, C3-7 cycloalkyl or C3-7 cycloalkyloxy substituted by 1 to 3 substituent(s) selected from the following groups of (1) to (40): (1) C1-6 alkyl, (2) C2-6 alkenyl, (3) C2-6 alkynyl, (4) C1-6 alkyloxy, (5) C1-6 alkyloxy-C1-6 alkyl, (6) C1-6 alkyloxy-C1-6 alkyloxy, (7) C2-6 alkenyloxy-C1-6 alkyl, (8) C1-6 alkyl substituted by 1 to 3 hydroxy, (9) C1-6 alkyl substituted by 1 to 3 halogen atom(s), (10) C1-6 alkylthio, (11) C1-6 alkylthio-C1-6 alkyl, (12) C1-6 alkylthio-C1-6 alkyloxy, (13) C2-6 alkenylthio-C1-6 alkyl, (14) C1-6 alkylsulfonyl, (15) halogen atom, (16) trihalomethyl, (17) cyano, (18) nitro, (19) amino, (20) hydroxy, (21) C3-7 cycloalkyl, (22) C3-7 cycloalkyloxy, (23) C3-7 cycloaikyl-C1-6 alkyl, (24) C3-7 cycloalkyloxy-C1-6 alkyl, (25) phenyl, (26) phenyloxy, (27) phenyl-C1-6 alkyl, (28) phenyl-C2-6 alkenyl, (29) phenyl-C2-6 alkynyl, (30) phenyloxy-C1-6 alkyl, (31) phenyloxy-C2-6 alkenyl, (32) phenyloxy-C2-6 alkynyl, (33) furyl, (34) furyloxy, (35) furyl-C1-6 alkyl, (36)furyloxy-C1-6 alkyl, (37) thienyl, (38) thienyloxy, (39) thienyl-C1-6 alkyl or (40) thienyloxy-C1-6 alkyl whereinthe above mentioned phenyl, furyl, thienyl and cycloalkyl may be substituted by 1 to 3 substituents selected from C1-6 alkyl, C1-6 alkyloxy, C1-6 alkyloxy-C1-6 alkyl, nitro, halogen, trihalomethyl, amino and hydroxy; or
      (v) furyl, furyloxy, thienyl, thienyloxy, naphthyl, naphthyloxy, phthalanyl or phthalanyloxy substituted by 1 to 3 substituents selected from the following groups: (1) C1-6 alkyl, (2) C2-6 alkenyl, (3) C2-6 alkynyl, (4) C1-6 alkyloxy, (5) C1-6 alkyloxy-C1-6 alkyl, (6) C1-6 alkyloxy-C1-6 alkyloxy, (7) C2-6 alkenyloxy-C1-6 alkyl, (8) C1-6 alkyl substituted by 1 to 3 hydroxy, (9) C1-6 alkyl substituted by 1 to 3 halogen atom(s), (10) C1-6 alkylthio, (11) C1-6 alkylthio-C1-6 alkyl, (12) C1-6 alkylthio-C1-6 alkyloxy, (13) C2-6 alkenylthio-C1-6 alkyl, (14) C1-6 alkylsulfonyl, (15) halogen atom, (16) trihalomethyl, (17) cyano, (18) nitro, (19) amino, (20) hydroxy, (21) C3-7 cycloalkyl, (22) C3-7 cycloalkyloxy, (23) C3-7 cycloalkyl-C1-6 alkyl, (24) C3-7 cycloalkyloxy-C1-6 alkyl, (25) phenyl, (26) phenyloxy, (27) phenyl-C1-6 alkyl, (28) phenyl-C2-6 alkenyl, (29) phenyl-C2-6 alkynyl, (30) phenyloxy-C1-6 alkyl, (31) phenyloxy-C2-6 alkenyl, (32) phenyloxy-C2-6 alkynyl, (33) furyl, (34) furyloxy, (35) furyl-C1-6 alkyl, (36) furyloxy-C1-6 alkyl, (37) thienyl, (38) thienyloxy, (39) thienyl-C1-6 alkyl or (40) thienyloxy-C1-6 alkyl whereinhe above mentioned phenyl, furyl, thienyl and cycloalkyl may be substituted by 1 to 3 substituent(s) selected from C1-6 alkyl, C1-6 alkyloxy, C1-6 alkyloxy-C1-6 alkyl, nitro, halogen, trihalomethyl, amino and hydroxy;
   R^{5D} is a hydrogen atom or C1-6 alkyl;
      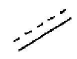 is a double bond or a single bond
   with the proviso that R^{2D} is a hydrogen atom, C1-6 alkylene represented by R^{3D} may be substituted by one hydroxy,
   a salt thereof, an N-oxide thereof or a solvate thereof, or a prodrug thereof, or a cyclodextrin clathrate thereof.
(E) In the pamphlet of WOOO/015608, it is described that the compound represented by the following formula (IE) has EP₄ agonistic activity. As well, the definition of each group of the compound represented by formula (IE) is described in the pamphlet of WO00/015608 in detail. Accordingly, EP₄ agonist of the present invention includes the compound represented by formula (IE): 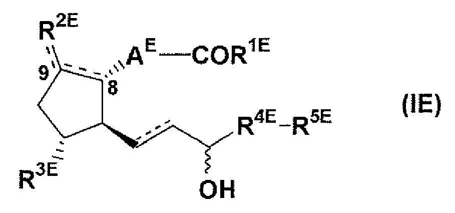 wherein A^{E} is C2-8 alkylene, C2-8 alkenylene, C1-4 alkylene-phenylene or C2-4 alkenylene-phenylene,
   R^{1E} is hydroxy, C1-6 alkyloxy, C1-6 alkyloxy-C1-6 alkyloxy, HO-C1-6 alkyloxy, or a formula of NR^{6E}R^{7E} wherein R^{6E} and R^{7E} are each independently hydrogen atom or C1-4 alkyl,
   R^{2E} is an oxygen atom, a halogen atom or R^{8E}-COO- wherein R^{8E} is hydrogen atom, C1-4 alkyl, phenyl or phenyl(C1-4 alkyl), C1-4 alkyloxy, HOOC-C1-4 alkyl, C1-4 alkyloxy-carbonyl-C1-4 alkyl, HOOC-C2-4 alkenyl, or C1-4 alkyloxy-carbonyl-C2-4 alkenyl,
   R^{3E} is a hydrogen atom or hydroxy,
   R^{4E} is C1-4 alkylene,
   R^{5E} is phenyl substituted by the following groups of i) to iv):
      i) 1 to 3 selected from (1) C1-4 alkyloxy-C1-4 alkyl, (2) C2-4 alkenyloxy-C1-4 alkyl, (3) C2-4 alkynyloxy-C1-4 alkyl, (4) C3-7 cycloalkyloxy-C1-4 alkyl, (5) C3-7 cycloalkyl(C1-4 alkyloxy)-C1-4 alkyl, (6) phenyloxy-C1-4 alkyl, (7) phenyl-C1-4 alkyloxy-C1-4 alkyl, (8) C1-4 alkylthio-C1-4 alkyl, (9) C2-4 alkenylthio-C1-4 alkyl, (10) C2-4 alkynylthio-C1-4 alkyl, (11) C3-7 cycloalkylthio-C1-4 alkyl, (12) C3-7 cycloalkyl(C1-4 alkylthio)-C1-4 alkyl, (13) phenylthio-C1-4 alkyl or (14) phenyl-C1-4 alkylthio-C1-4 alkyl,
      ii) (1) C1-4 alkyloxy-C1-4 alkyl and C1-4 alkyl, (2) C1-4 alkyloxy-C1-4 alkyl and C1-4 alkyloxy, (3) C1-4 alkyloxy-C1-4 alkyl and hydroxy, (4) C1-4 alkyloxy-C1-4 alkyl and halogen atom, (5) C1-4 alkylthio-C1-4 alkyl and C1-4 alkyl, (6) C1-4 alkylthio-C1-4 alkyl and C1-4 alkyloxy, (7) C1-4 alkylthio-C1-4 alkyl and hydroxy or (8) C1-4 alkylthio-C1-4 alkyl and halogen atom,
      iii) (1) halo-C1-4 alkyl or (2) hydroxy-C1-4 alkyl or
      iv) C1-4 alkyl and hydroxy;
         ---- is independently a single bond or a double bond, wherein continuous double bonds are not formed wherein when R^{2E} is R^{8E}-COO-, R^{1E} is C1-6 alkyloxy, C1-6 alkyloxy-C1-6 alkyloxy or HO-C1-6 alkyloxy and the bond in the 8-9 position is a double bond,
      a salt thereof, an N-oxide thereof or a solvate thereof, or a prodrug thereof, or a cyclodextrin clathrate thereof.
(F) In the pamphlet of WO01/149661, it is described that the compound represented by the following formula (IF) has EP₄ agonistic activity. As well, the definition of each group of the compound represented by formula (IF) is described in the pamphlet of WO01/149661 in detail. Accordingly, EP₄ agonist of the present invention includes the compound represented by formula (IF) : 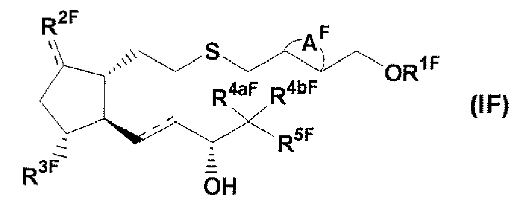 wherein --A^{F}-- is absent or --A^{F}-- is methylene or ethylene, R^{1F} is a hydrogen atom, C1-6 alkyl, phenyf-C1-6 alkyl, C2-6 alkanoyl or phenyl-C2-6 alkanoyl,
   R^{2F} is an oxygen atom or a halogen atom,
   R^{3F} is a hydrogen atom or hydroxy,
   R^{4aF} and R^{4bF} are each independently hydrogen atom or C1-4 alkyl,
   R^{5F} is phenyl substituted with the following group of i) to iv):
      i) 1 to 3 selected from (1) C1-4 alkyloxy-C1-4 alkyl, (2) C2-4 alkenyloxy-C1-4 alkyl, (3) C2-4 alkynyloxy-C1-4 alkyl, (4) C3-7 cycloalkyloxy-C1-4 alkyl, (5) C3-7 cycloalkyl(C1-4 alkyloxy)-C1-4 alkyl, (6) phenyloxy-C1-4 alkyl, (7) phenyl-C1-4 alkyloxy-C1-4 alkyl, (8) C1-4 alkylthio-C1-4 alkyl, (9) C2-4 alkenylthio-C1-4 alkyl, (10) C2-4 alkynylthio-C1-4 alkyl, (11) C3-7cycloalkylthio-C1-4 alkyl, (12) C3-7 cycloalky(C1-4 alkylthio)-C1-4 alkyl, (13) phenylthio-C1-4 alkyl or (14) phenyl-C1-4 alkylthio-C1-4 alkyl,
      ii) (1) C1-4 alkyloxy-C1-4 alkyl and C1-4 alkyl, (2) C1-4 alkyloxy-C1-4 alkyl and C1-4alkyloxy, (3) C1-4 alkyloxy-C1-4 alkyl and hydroxy, (4) C1-4 alkyloxy-C1-4 alkyl and a halogen atom, (5) C1-4 alkylthio-C1-4 alkyl and C1-4 alkyl, (6) C1-4 alkylthio-C1-4 alkyl and C1-4 alkyloxy, (7) C1-4 alkylthio-C1-4 alkyl and hydroxy or (8) C1-4 alkylthio-C1-4 alkyl and halogen atom,
      iii) haloalkyl or hydroxy-C1-4 alkyl, or
      iv) C1-4 alkyl and hydroxy;
         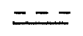 is a single bond or a double bond,
      a salt thereof, an N-oxide thereof or a solvate thereof, or a prodrug thereof, or a cyclodextrin clathrate thereof.
(G) In the pamphlet of WO01/166518, it is described that the compound represented by the following formula (IG) has EP₄ agonistic activity. As well, the definition of each group of the compound represented by formula (IG) is described in the pamphlet of WO01/166518 in detail. Accordingly, EP₄ agonist of the present invention includes the compound represented by formula (IG): 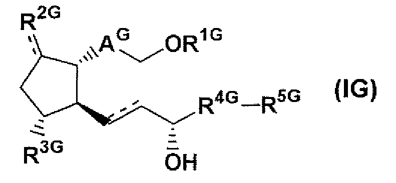 wherein A^{G} is C2-8 alkylene, C2-8 alkenylene, C1-4 alkylene-phenylene or C2-4 alkenylene-phenylene,
   R^{1G} is a hydrogen atom, C1-6 alkyl, phenyl-C1-6 alkyl, C2-6 alkanoyl, phenyl-C2-6 alkanoyl,
   R^{2G} is an oxygen atom or a halogen atom,
   R^{3G} is a hydrogen atom or hydroxy,
   R^{4G} is C1-4 alkylene,
   R^{5G} is phenyl substituted with the following group of i) to iv):
      i) 1 to 3 selected from (1) C1-4 alkyloxy-C1-4 alkyl, (2) C2-4 alkenyloxy-C1-4 alkyl, (3) C2-4 alkynyloxy-C1-4 alkyl, (4) C3-7 cycloalkyloxy-C1-4 alkyl, (5) C3-7 cycloalkyl(C1-4 alkyloxy)-C1-4 alkyl, (6) phenyloxy-C1-4 alkyl, (7) phenyl-C1-4 alkyloxy-C1-4 alkyl, (8) C1-4 alkylthio-C1-4 alkyl, (9) C2-4 alkenylthio-C1-4 alkyl, (10) C2-4 alkynylthio-C1-4 alkyl, (11) C3-7 cycloalkylthio-C1-4 alkyl, (12) C3-7 cycloalkyl(C1-4 alkylthio)-C1-4 alkyl, (13) phenylthio-C1-4 alkyl or (14) phenyl-C1-4 alkylthio-C1-4 alkyl,
      ii) (1) C1-4 alkyloxy-C1-4 alkyl and C1-4 alkyl, (2) C1-4 alkyloxy-C1-4 alkyl and C1-4 alkyloxy, (3) C1-4 alkyloxy-C1-4 alkyl and hydroxy, (4) C1-4 alkyloxy-C1-4 alkyl and halogen atom, (5) C1-4 alkylthio-C1-4 alkyl and C1-4 alkyl, (6) C1-4 alkylthio-C1-4 alkyl and C1-4 alkyloxy, (7) C1-4 alkylthio-C1-4 alkyl and hydroxy or (8) C1-4 alkylthio-C1-4 alkyl and halogen atom,
      iii) (1) halo C1-4 alkyl or (2) hydroxyl C1-4 alkyl, or
      iv) C1-4 alkyl and hydroxy;
         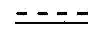 is a single bond or double bond,
      a salt thereof, an N-oxide thereof or a solvate thereof, or a prodrug thereof, or a cyclodextrin clathrate thereof.
(H) In the pamphlet of WO2004/065365, it is described that the compound represented by the following formula (IH) has EP₄ agonistic activity. As well, the definition of each group of the compound represented by formula (IH) is described in the pamphlet of WO2004/065365 in detail. Accordingly, EP₄ agonist of the present invention includes the compound represented by formula (IH): 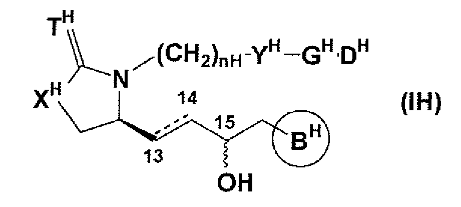 wherein 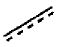 is a single bond or a double bond,
   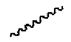 is α-configuration, β-configuration or mixture thereof having an optical mixing ratio,
   D^{H} is -COOR^{1H} or tetrazolyl,
   R^{1H} is a hydrogen atom or C1-4 alkyl,
   G^{H} is ringA^{H} or C1-4 alkylene, 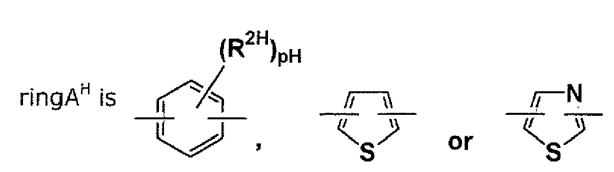 wherein R^{2H} is a halogen atom, C1-4 alkyl or C1-4 alkoxy, pH is 0 or an integer of 1 to 4, when pH is more than 2, each of R^{2H} is same or different.,
   Y^{H} is a bond or -S-,
   T^{H} is an oxygen atom or a sulfur atom,
   X^{H} is -CH₂-, -O- or -S-,
   ringB^{H} is C3-7 cycloalkyl which may be have a substituent(s), 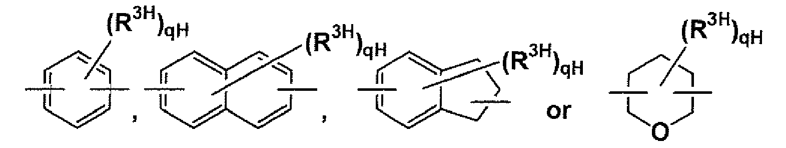 wherein R^{3H} is (1) a halogen atom, (2) C1-4 alkyl which may besubstituted by 1 to 5 halogen atom(s), (3) C1-4 alkoxyl which may be substituted by 1 to 5 halogen atom(s), (4) C1-4 alkyl substituted by C1-4 alkoxy, (5) phenyl or (6) 3 to 15 membered mono-, bi- or tri-heterocyclic ring which comprises 1-4 hetero atom(s) selected from oxygen, nitrogen and sulfur, and may be saturated partially or fully, (5) phenyl or (6) heterocyclic ring in R^{3H} may be substituted by 1 to 3 substituent(s) selected form (a) halogen atom, (b) C1-4 alkyl, (c) C1-4 alkoxy and/or (d) nitro, qH is 0 or an integer of 1 to 5, when qH is more than 2, each R^{3H} may be same or different,
   nH is an integer of 1 to 4.,
   a salt thereof, an N-oxide thereof or a solvate thereof, or a prodrug thereof, or a cyclodextrin clathrate thereof.
(J) In the pamphlet of WO02/24647, it is described that the compound represented by the following formula (IJ) has EP₄ agonistic activity. As well, the definition of each group of the compound represented by formula (IJ) is described in the pamphlet of WO02/24647 in detail. Accordingly, EP₄ agonist of the present invention includes the compound represented by formula (IJ): 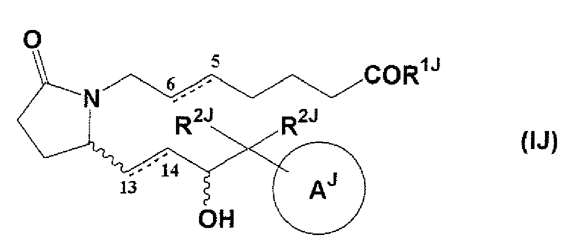 wherein, R^{IJ} is hydroxyl, C1-4 alkoxy, NHSO₂-C1-4 alkyl, or NHCO-phenyl, A^{J} is benzene or thiophene, two R^{2J} are hydrogen atom(s) or fluorine atom(s) simultaneously,
   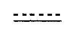 is a single bond or a double bond, wherein when R^{2J} is fluorine atom, A^{J} is only benzene,
   a salt thereof, an N-oxide thereof or a solvate thereof, or a prodrug thereof, or a cyclodextrin clathrate thereof.
(K) In the pamphlet of WO02/042268, it is described that the compound represented by the following formula (IK) has EP₄ agonistic activity. As well, the definition of each group of the compound represented by formula (IK) is described in the pamphlet of WO02/042268 in detail. Accordingly, EP₄ agonist of the present invention includes the compound represented by formula (IK): 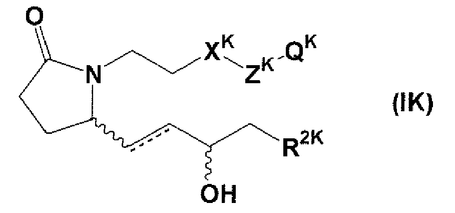 wherein, the dotted line is a bond or no bond,
   X^{K} is -CH₂- or -O-,
   Z^{K} is -(CH₂)₃-, thienyl, thiazolyl or phenyl, provided that X^{K} is O, then Z^{K} is phenyl,
   Q^{K} is carboxyl, C1-4 alkoxycarbonyl or tetrazolyl,
   R^{2K} is -Ar^{K} or -Ar^{1K}-V^{K}-Ar^{2K}- ,
   V^{K} is a bond, -O-, -OCH₂- or -CH₂O-,
   Ar^{K} is partially saturated, fully saturated or fully unsaturated 5 to 8 membered ring which may be substituted by 1 to 4 heteroatoms selected voluntarily from oxygen, sulfur and nitrogen, or bicyclic ring consisting of two fused partially saturated, fully saturated or fully unsaturated 5 to 6 membered rings, which may be substituted by 1 to 4 heteroatoms selected voluntarily from oxygen, sulfur and oxygen, said partially or fully saturated ring or bicyclic ring which may be substituted by 1 or 2 oxo groups substituted on carbon or 1 or 2 oxo groups substituted on sulfur,
   Ar^{1K} and Ar^{2K} are each independently a partially saturated, fully saturated or fully unsaturated 5 to 8 membered ring which may be substituted by 1 to 4 heteroatoms selected voluntarily from oxygen, sulfur or nitrogen, said partially or fully saturated ring which may be substituted by 1 or 2 oxo groups substituted on carbon or 1 or 2 oxo groups substituted on sulfur,
   said Ar^{K} moiety may be substituted on carbon or nitrogen, on one ring if the moiety is monocyclic, or one or both rings if the moiety is bicyclic, with up to three substituents selected voluntarily from; (1) hydroxyl, (2) halogen atom, (3) carboxyl, (4) C1-7 alkoxy, (5) C1-4 alkoxy C1-4 alkyl, (6) C1-7 alkyl, (7) C2-7 alkenyl, (8) C3-7 cycloalkyl, (9) C3-7 cycloalkyl C1-4 alkyl, (10) C3-7 cycloalkyl C1-4 alkanoyl, (11) formyl, (12) C1-8 alkanoyl, (13) C1-6 alkanoyl C1-4 alkyl, (14) C1-4 alkanoylamino, (15) C1-4 alkoxycarbonylamino, (16) hydroxysulfonyl, (17) aminocarbonylamino or mono-N-, di-N,N-, di-N,N'-, or tri-N,N,N'-aminacarbonyl substituted C1-4 alkyl, (18) sulfonamide, (19) C1-4 alkylsulfonamide, (20) amino, (21) mono-N- or di-N,N-C1-4 alkylamino, (22) carbamoyl, (23) mono-N- or diN,N-C1-4 alkylcarbamoyl, (24) cyano, (25) thiol, (26) C1-6 alkylthio, (27) C1-6 alkylsulfinyl, (28) C1-4 alkylsulfonyl, (29) mono-N- or di-N,N-C1-4 alkylaminosulfinyl, wherein said alkyl and alkoxy substituents in the definition of Ar^{K} may be substituted on carbon with up to three fluorine atom,
   said Ar^{1K} and Ar^{2K} moieties may be substituted on carbon or nitrogen with up to three substituents selected from; (1) hydroxyl, (2) a halogen atom, (3) carboxyl, (4) C1-7 alkoxy, (5) C1-4 alkoxy C1-4 alkyl, (6) C1-7 alkyl, (7) C2-7 alkenyl, (8) C3-7 cycloalkyl, (9) C3-7 cycloalkyl C1-4 alkyl, (10) C3-7 cycloalkyl C1-4 alkanoyl, (11) formyl, (12) C1-8 alkanoyl, (13) C1-6 alkanoyl C1-4 alkyl, (14) C1-4 alkanoylamino, (15) C1-4 alkoxycarbonylamino, (16) hydroxysulfonyl, (17) aminocarbonylamino or mono-N-, di-N,N-, di-N,N'-, or tri-N,N,N'-aminocarbonyl substituted C1-4 alkyl, (18) sulfonamide, (19) C1-4 alkylsulfonamide, (20) amino, (21) mono-N- or di-N,N-C1-4 alkylamino, (22) carbamoyl, (23) mono-N- or diN,N-C1-4 alkylcarbamoyl, (24) cyano, (25) thiol, (26) C1-6 alkylthio, (27) C1-6 alkylsulfinyl, (28) C1-4 alkylsulfonyl, (29) mono-N- or di-N,N-C1-4 alkylaminosulfinyl, wherein said alkyl and alkoxy substituents in the definition of Ar^{1K} and Ar^{2K} may be substituted on carbon with up to three fluorine atom, provided that (a) when X^{K} is -(CH₂)- and Z^{K} is -(CH₂)₃-, the R^{2K} is not thienyl, phenyl or phenyl monosubstituted with chlorine, fluorine, phenyl, methoxy, trifluoromethoxy or C1-4 alkyl, (b) when X^{K} is -(CH₂)-, Z^{K} is -(CH₂)₃-, and Q^{K} is carboxyl or C1-4 alkoxycarbonyl, the R^{2K} is not (i) C5-7 cycloalkyl or (ii) phenyl, thienyl or furyl each of which may be optionally monosubstituted or disubstituted by one or two substituents selected from (1) halogen atom or (2) C1-3 alkyl which may be substituted by one or more halogen atoms or C1-4 alkoxy.,
   a salt thereof, an N-oxide thereof or a solvate thereof, or a prodrug thereof, or a cylcodextrin clathrate thereof.
(L) In the pamphlet of WO03/008377 it is described that the compound represented by the following formula (IL) has EP₄ agonistic activity. As well, the definition of each group of the compound represented by formula (IL) is described in the pamphlet of WO03/008377. Accordingly, EP₄ agonist of the present invention includes the compound represented by formula (IL): 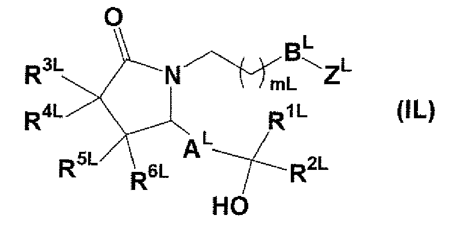 wherein, A^{L} is -CH₂-CH₂- or -CH=CH-,
   B^{L} is a single bond, aryl or heteroaryl,
   Z^{L} is -C(O)OR^{L}, -C(O)NR'^{L}R"^{L}, -C(O)NSO₂R'^{L}, -PR'^{L}(O)(OR'^{L}), - PO(OR'^{L})₂ or tetrazol-5-yl wherein, R'^{L} and R"^{L} are each independently hydrogen atom or C1-6 alkyl,
   mL is 1,2,3,4,5 or 6,
   R^{1L} is alkyl, alkenyl, alkynyl, cycloalkylalkyl, heterocyclylalkyl, aryl, arylalkyl, heteroaryl, when B^{L} is aryl or heteroaryl and R^{3L}, R^{4L}, R^{5L} and R^{6L} are not simultaneously a hydrogen atom, or R^{1L} is heterocyclylalkyl, aryl, arylalkyl, heteroaryl, when B^{L} is a single bond and R^{3L}, R^{4L}, R^{5L} and R^{6L} are simultaneously a hydrogen atom,
   R^{2L} is a hydrogen atom, C1-6 alkyl, C1-6 alkenyl or C1-6 alkynyl,
   R^{3L}, R^{4L}, R^{5L} and R^{6L} are each independently hydrogen atom or C1-6 alkyl, R^{3L} and R^{4L}, R^{5L} and R^{6L} or R^{3L} and R^{5L} taken together with the atom to which they are attached may form C3-7 alkyl ring.,
      a salt thereof, an N-oxide thereof or a solvate thereof, or a prodrug thereof, or a cyclodextrin clathrate thereof.
(M) In the pamphlet of WO03/035064, it is described that the compound represented by the following formula (IM) binds to EP₄. As well, the definition of each group of the compound represented by formula (IM) is described in the pamphlet of WO03/035064. Accordingly, EP₄ agonist of the present invention includes the compound represented by formula (IM): 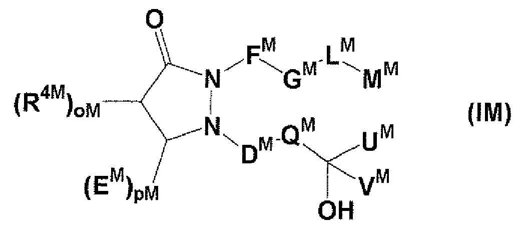 wherein, each R^{4M} is independently a hydrogen atom, alkyl whicn may be substituted, carbocyclic aryl whicn may be substituted or heteroaromatic whicn may be substituted,
   E^{M} is a hydrogen atom, hydroxyl, alkoxy which may be substituted or alkylthio whicn may be substituted,
   oM and pM are each independently 0, 1 or 2, the sum of oM and pM is at least 1,
   F^{M} is -(CH₂)_{nM} wherein nM is an integer of from 1 to 6,
   G^{M} is -C≡C-, -CH=CH-, -CH₂-, carbocyclic aryl whicn may be substituted or heteroaromatic whicn may be substituted,
   L^{M} is (CH₂)_{n'M} wherein n'M is an integer of from 0 to 3,
   M^{M} is COX^{M}, SO₂X^{M} wherein, X^{M} is OR'^{M} or NHR'^{M}, R'^{M} is H or alkyl whicn may be substituted, tetrazol whicn may be substituted, NO₂, NHSO₂R^{M} or NHC(O)R^{M} wherein, R^{M} is H, alkyl whicn may be substituted,
   D^{M} is (CH₂)_{n"M} wherein, n"M is an integer of from 0 to 2,
   Q^{M} is (CH2)_{n'"M} wherein, n"'M is 0 or 1, -CH=CH- or carbocyclic aryl whicn may be substituted, preferably phenyl whicn may be substituted,
   U^{M} and V^{M} are each independently alkyl whicn may be substituted, alkenyl whicn may be substituted, alkynyl whicn may be substituted, carbocyclic aryl which may be substituted, or heteroaromatic whicn may be substituted wherein the compound wherein G^{M} is CH₂, n'^{M} is 3, E^{M} is a hydrogen atom and pM is 2, R4^{M} is a hydrogen atom and oM is 2, n"M is 2, n"'M is 0, V^{M} is alkyl, is excepted,
      a salt thereof, an N-oxide thereof or a solvate thereof, a prodrug thereof, or a cyclodextrin clathrate thereof.
(N) In the pamphlet of WO03/053923, it is described that the compound represented by the following formula (IN) binds to EP₄. As well, the definition of each group of the compound represented by formula (IN) is described in the pamphlet of WO03/053923. Accordingly, EP₄ agonist of the present invention includes the compound represented by formula (IN): 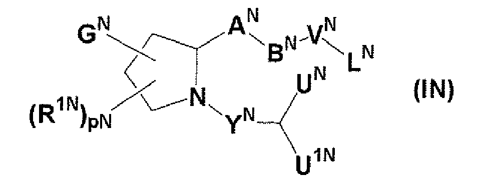 wherein, each R^{1N} is independently a hydrogen atom, alkyl whicn may be substituted, alkenyl whicn may be substituted, alkynyl whicn may be substituted, heteroalkyl whicn may be substituted, heteroalkenyl whicn may be substituted, heteroalkynyl whicn may be substituted, carbocyclic aryl whicn may be substituted, aralkyl whicn may be substituted, heteroalicyclic whicn may be substituted, heteroaryl whicn may be substituted, heteroarylalkyl whicn may be substituted or heteroalicyclicalkyl whicn may be substituted,
   G^{N} is oxo, a halogen atom, alkyl whicn may be substituted, alkoxy, hydroxyl, carboxylate which may be substituted, alkylcarboxylate ester whicn may be substituted,
   P^{N} is an integer of from 0 to 4,
   Y^{N} is (CR^{2N}R^{3N})_{qN} which may include 0 or 1 C=C double bond wherein qN is an integer of from 1 to 6, R^{2N} and R^{3N} are each independently a hydrogen atom, alkyl whicn may be substituted, alkenyl whicn may be substituted, alkynyl whicn may be substituted, hydroxyl, a halogen atom or alkoxy whicn may be substituted,
   U^{N} and U^{1N} are each independently a hydrogen atom, hydroxyl or substituted alkyl whicn may be substituted,
   A^{N} is O, S, (CR^{2N}R^{3N})_{q'N} wherein q'N is an integer of from 1 to 6,
   B^{N} is (CR^{2N}R^{3N})_{nN} or a single bond,
   A^{N} and B^{N} take together to form 1,2-vinylene or ethynylene whicn may be substituted,
   V^{N} is (CR^{2N}R^{3N})_{mN}, divalent aryl whicn may be substituted or divalent heteroaryl whicn may be substituted,
   L^{N} is C(O)Z^{N},
   Z^{N} is hydroxyl, alkyl whicn may be substituted, alkenyl whicn may be substituted, alkynyl whicn may be substituted, heteroalkyl whicn may be substituted, heteroalkenyl whicn may be substituted, heteroalkynyl whicn may be substituted, amino whicn may be substituted, NR^{4N}R^{5N}, cycloalkyl whicn may be substituted, heterocycloalkyl whicn may be substituted, carbocyclic aryl whicn may be substituted, heteroaryl whicn may be substituted, arylalkyl whicn may be substituted or heteroarylalkyl whicn may be substituted,
   nN is an integer of from 0 to 3,
   mN is an integer of from 1 to 6,
   R^{4N} and R^{5N} are each independently a hydrogen atom, alkyl whicn may be substituted, cycloalkyl whicn may be substituted, heterocycloalkyl whicn may be substituted, alkenyl whicn may be substituted, alkynyl whicn may be substituted, heteroalkyl whicn may be substituted, heteroalkenyl whicn may be substituted, heteroalkynyl whicn may be substituted, carbocyclic aryl whicn may be substituted, heteroaryl whicn may be substituted, arylalkl whicn may be substituted or heteroarylalkl whicn may be substituted, or R^{4N} and R^{5N} take together to be heterocycloalkyl,
      a salt thereof, an N-oxide thereof or a solvate thereof, or a prodrug thereof, or a cylcodextrin clathrate thereof.
(P) In the pamphlet of WO03/103664, it is described that the compound represented by the following formula (IP) has EP₄ agonistic activity. As well, the definition of each group of the compound represented by formula (IP) is described in the pamphlet of WO03/103664. Accordingly, EP₄ agonist of the present invention includes the compound represented by formula (IP): 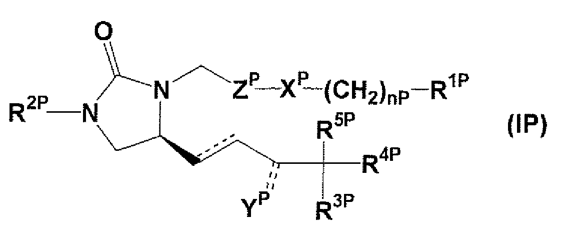 wherein X^{P} is a single bond, an oxygen atom or a sulfur atom,
   Y^{P} is = O or -OH,
   R^{1P} is hydroxyl, CN, (CH₂)_{pP}CO₂R^{6P}, (CH₂)_{nP}SO₃R^{6P}, -CF₂SO₂NH₂, - SO₂NH₂, -CHNHSO₂R^{2P}-, -SO₂NHCOR^{6P}, -PO(OH)₂, CONHPO₂R^{6P}, CONHR^{8P}, C1-4 alkoxy, -(CH₂)_{nP}NR^{6P}R^{7P}, hydroxymethylketone or -(CH₂)_{nP}heterocyclyl, said heterocyclyl unsubstituted or substituted with 1 to 3 R^{aP} and may contain an acidic hydrogen atom,
   R^{2P} is a hydrogen atom, C6-10 aryl or C1-4 alkyl,
   R^{3P} and R^{4p} are each independently a hydrogen atom, a halogen atom or C1-6 alkyl,
   R^{5P} is (CH₂)_{mP}C6-10aryl, (CH₂)_{mP}C5-10heteroaryl, (CH₂)_{mP}C3-10heterocycloalkyl or (CH₂)_{mP}C3-10cycloalkyl, said cycloalkyl, heterocycloalkyl, aryl or heteroaryl unsubstituted or substituted with 1 to 3 R^{aP} ,
   R^{6P} and R^{7P} are a hydrogen atom or C1-4 alkyl,
   R^{8P} is a hydrogen atom or sulfonyl,
   Z^{P} is (C(R^{bP})₂)_{nP,} 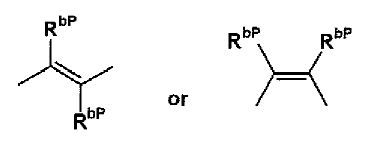 each R^{bP} is independently a hydrogen atom, a halogen atom, C1-6 alkyl or C3-6 cycloalkyl,
   R^{aP} is C1-6 alkoxy, C1-6 alkyl, CF₃, nitro, amino, cyano, C1-6 alkylamino or a halogen atom,
   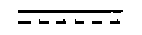 is a double bond or a single bond,
   pP is 1 to 3,
   nP is 0 to 4,
   mP is 0 to 8],
   a salt thereof, an N-oxide thereof or a solvate thereof, or a prodrug thereof, or a cyclodextrin clathrate thereof.
(Q) In the pamphlet of WO03/007941, it is described that the compound represented by the following formula (IQ) has EP₄ agonistic activity. As well, the definition of each group of the compound represented by formula (IQ) is described in the pamphlet of WO03/007941. Accordingly, EP₄ agonist of the present invention includes the compound represented by formula (IQ): 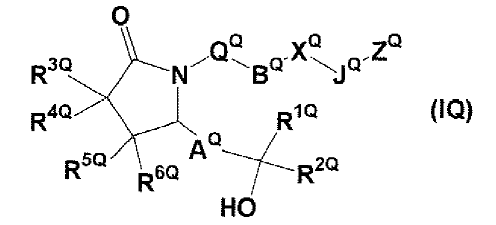 wherein Q^{Q} is CH_{2,} oxygen atom,
   B^{Q} is -CH₂-, -(CH₂)₂-, -(CH₂)₃-, -(CH₂)₄-, -(CH₂)₅-, -CH=CH-, -CH₂-CH=CH-, -CH=CH-CH₂- or -CH₂-CH=CH-CH₂-, wherein when B^{Q} is -CH=CH-, or -CH=CH-CH₂-, then Q^{Q} is CH_{2,}
   X^{Q} is -NR^{aQ}- wherein R^{aQ} is a hydrogen atom, a halogen atom, C1-6 alkyl, C1-6 acyl, -O-, -S-, -SO-, -SO₂- or a single bond, wherein that X^{Q} is a single bond, then Q^{Q} is an oxygen atom,
   J^{Q} is -(CR^{bQ}R^{cQ})_{nQ}- (wherein, nQ is an integer of from 1 to 4, R^{bQ} and R^{cQ} are both hydrogen atom, or one or two of R^{bQ} and R^{cQ} are lower alkyl and the remainder is a hydrogen atom, or R^{bQ} and R^{cQ} if attached to the same carbon atom form a C2-5 polymethylene) or -CH₂-CH=CH-,
   A^{Q} is -CH₂-CH₂-, -CH=CH- or -C≡C-,
   Z^{Q} is CH₂OH, -C(O)OR'^{Q}, -C(O)NR'^{Q}R"^{Q}, -C(O)NSO₂R'^{Q}, -P(C1-6 alkyl)(O)(OR'^{Q}), -PO(OR'^{Q})₂ or tetrazol-5-yl wherein R'^{Q} and R"^{Q} are each independently hydrogen atom or C1-6 alkyl,
   nQ is 1, 2, 3 or 4,
   R^{1Q} is -(CH₂)_{pQ}R^{7Q} or -(CH₂)_{qQ}OR^{8Q} wherein R^{7Q} and R^{8Q} are each independently C1-6 alkyl, halo C1-6 alkyl, C3-6 cycloalkyl, heterocyclyl, aryl or heteroaryl, pQ and qQ are each independently 0, 1, 2, 3, 4 or 5,
   R^{2Q} is a hydrogen atom, C1-6 alkyl, C1-6 alkenyl, C1-6 alkynyl,
   R^{3Q}, R^{4Q}, R^{5Q} and R^{6Q} are each independently a hydrogen atom or C1-6 alkyl,
   a salt thereof, an N-oxide thereof or a solvate thereof, or a prodrug thereof, or a cyclodextrin clathrate thereof.
(R) In the pamphlet of WO03/074483, it is described that the compound represented by the following formula (IR) has EP₄ agonistic activity. As well, the definition of each group of the compound represented by formula (IR) is described in the pamphlet of WO03/074483. Accordingly, EP₄ agonist of the present invention includes the compound represented by formula (IR): 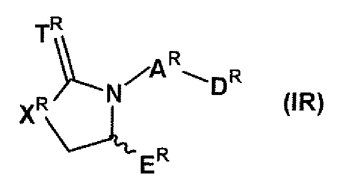 wherein T^{R} is (1) an oxygen atom, or (2) a sulfur atom,
   X^{R} is (1) -CH₂-, (2) -O-, or (3) -S-,
   A^{R} is A^{1R} or A^{2R},
   A^{1R} is (1) C2-8 straight chain alkylene which may be substituted by 1 or 2 C1-4 alkyl, (2) C2-8 straight chain alkenylene which may be substituted by 1 or 2 C1-4 alkyl, or (3) C2-8 straight chain alkynylene which may be substituted by 1 or 2 C1-4 alkyl,
   A^{2R} is -G^{1R}-G^{2R}-G^{3R}-,
   G^{1R} is (1) C1-4 straight chain alkylene which may be substituted by 1 or 2 C1-4 alkyl, (2) C2-4 straight chain alkenylene which may be substituted by 1 or 2 C1-4 alkyl, (3) C2-4 straight chain alkynylene which may be substituted by 1 or 2 C-4 alkyl,
   G^{2R} is (1) -Y^{R}-, (2) -ring1^{R}-, (3) -Y^{R}-ring^{1R}-, (4) -ring^{1R}-Y^{R}-, or (5) - Y^{R}-C1-4alkylene-ring^{1R}-,
   Y^{R} is (1) -S-, (2)-SO-, (3) -SO₂-, (4) -O-, or (5) -NR^{1R}-,
   R^{1R} is (1) a hydrogen atom, (2) C1-10 alkyl, or (3) C2-10 acyl,
   G^{3R} is (1) a bond, (2) C1-4 straight chain alkylene which may be substituted by 1 or 2 C1-4 alkyl, (3) C2-4 straight chain alkenylene which may be substituted by 1 or 2 C1-4 alkyl, or (4) C2-4 straight chain alkynylene which may be substituted by 1 or 2 C1-4 alkyl,
   D^{R} is D^{1R} or D^{2R},
   D^{1R} is (1) -COOH, (2) -COOR^{2R}, (3) tetrazol-5-yl, or (4) - CONR^{3R}SO₂R^{4R},
   R^{2R} is (1) C1-10 alkyl, (2) phenyl, (3) C1-10 alkyl substituted by phenyl, or (4) biphenyl,
   R^{3R} is (1) a hydrogen atom, or (2) C1-10 alkyl,
   R^{4R} is (1) C1-10 alkyl, or (2) phenyl,
   D^{2R} is (1) -CH₂OH, (2) -CH₂OR^{5R}, (3) hydroxy, (4) -OR^{5R}, (5) formyl, (6) -CONR^{6R}R^{7R}, (7) -CONR^{6R}SO₂R^{8R}, (8) -CO-(NH-amino acid residue-CO)_{mR}-OH, (9) -O-(CO-amino acid residue-NH)_{mR}-H, (10) -COOR^{9R}, (11) - OCO-R^{10R}, (12) -COO-Z^{1R}-Z^{2R}-Z^{3R}, or (13) 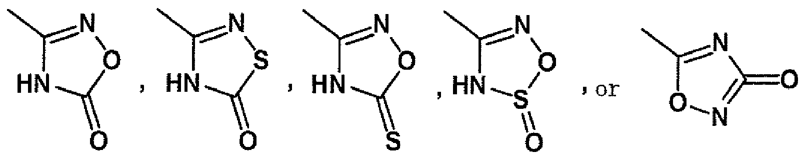
   R^{5R} is C1-10 alkyl,
   R^{6R} and R^{7R} are each independently (1) a hydrogen atom, or (2) C1-10 alkyl,
   R^{8R} is C1-10 alkyl substituted by phenyl,
   R^{9R} is (1) C1-10 alkyl substituted by biphenyl which may be substituted by 1 to 3 substituents selected from C1-10 alkl, C1-10 alkoxy and a halogen atom, or (2) biphenyl substituted by 1 to 3 substituents selected from C1-10 alkyl, C1-10 alkoxy, and a halogen atom,
   R^{10R} is (1) phenyl, or (2) C1-10 alkyl,
   mR is 1 or 2,
   Z^{1R} is (1) C1-15 alkylene, (2) C2-15 alkenlylene, or (3) C2-15 alkynylene,
   Z^{2R} is (1) -CO-, (2) -OCO-, (3) -COO-, (4) -CONR^{Z1R}-, (5) -NR^{Z2R}CO-, (6) -O-, (7) -S-, (8) -SO₂-, (9) -SO₂-NR^{Z2R}-, (10) -NR^{Z2R}SO₂-, (11) -NR^{Z3R}-, (12) -NR^{Z4R}CONR^{Z5R} - (13) -NR^{z6R}COO-, (14) -OCONR^{Z7R}-, or (15) -OCOO-,
   Z^{3R} is (1) hydrogen atom, (2) C1-15 alkyl, (3) C2-15 alkenyl, (4) C2-15 alkynyl, (5) ringZ^{R}, or (6) C1-10 alkoxy, C1-10 alkylthio, C1-10 alkyl-NR^{z8R}- or C1-10 alkyl substituted by ringZ^{R},
   ringZ^{R} is (1) optionally or fully saturated C3-15 mono-, bi-, tri-carbocyclic aryl or (2) optionally or fully saturated 3-15 membered mono-, bi-, tri-heterocyclic aryl containing 1-4 hetero atoms selected from an oxygen atom, a nitrogen atom, and a sulfur atom,
   R^{Z1R}, R^{Z2R}, R^{Z3R}, R^{Z4R}, R^{Z5R}, R^{Z6R}, R^{Z7R} and R^{Z8R} are each independently a hydrogen atom or C1-15 alkyl,
   R^{Z1R} and Z^{3R} taken together with the nitrogen atom which they attached may be 5-7 membered saturated mono-heterocyclic ring, the above described heterocyclic ring may further contain one hetero atom selected from oxygen atom, nitrogen atom and sulfur atom,
   RingZ^{R} and the saturated mono-heterocyclic ring formed by R^{Z1R} and Z^{3R} and the nitrogen atom to which they are attached may be substituted by 1 to 3 groups selected from following (1) to (4);
      (1) C1-15 alkyl, (2) C2-15 alkenyl, (3) C2-15 alkynyl, (4) C1-10 alkyl substituted by C1-10 alkoxy, C1-10 alkylthio, or C1-10 alkyl-NR^{Z9R}-;
   R^{Z9R} is a hydrogen atom, or C1-10 alkyl,
   E^{R} is E^{1R} or E^{2R},
   E^{1R} is 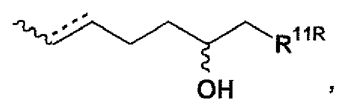
   R^{11R} is (1) C1-10 alkyl, (2) C1-10 alkylthio, (3) C1-10 alkyl substituted by C3-8 cycloalkyl, (4) C1-10 alkyl substituted by ring2^{R}, or (5) C1-10 alkyl substituted by -w^{1R}-W^{2R}-ring2^{R},
   W^{1R} is (1) -O-, (2) -S-, (3) -SO-, (4) -SO₂-, (5) -NR^{11-1R}-, (6) carbonyl, (7) -NR^{11-1R}SO₂-, (8) carbonylamino, or (9) aminocarbonyl,
   R^{11-1R} is (1) a hydrogen atom, (2) C1-10 alkyl, or (3) C2-10 acyl,
   W^{2R} is (1) a bond, or (2) C1-8 alkyl substituted by C1-4 alkyl, halogen atom, or hydroxy,
   E^{2R} is (1) U^{1R}-U^{2R}-U^{3R}, or (2) ring4^{R},
   U^{1R} is (1) C1-4 alkylene, (2) C2-4 alkenylene, (3) C2-4 alkynylene, (4) -ring3^{R}-, (5) C1-4 alkylene-ring3^{R}-, (6) C2-4 alkenylene-ring3^{R}-, or (7) C2-4 alkynylene-ring3^{R}-,
   U^{2R} is (1) a bond, (2) -CH₂-, (3) -CHOH-, (4) -O-, (5) -S-, (6) -SO-, (7) -SO₂- (8) -NR^{12R}-, (9) carbonyl, (10) -NR^{12R}SO₂-, (11) carbonylamino, or (12) aminocarbonyl,
   R^{12R} is (1) a hydrogen atom, (2) C1-10 alkyl, or (3) C2-10 acyl,
   U^{3R} is (1) C1-8 alkyl which may be substituted by 1 to 3 substitutents selected from C1-10 alkyl, a halogen atom, hydroxyl, alkoxy, alkylthio, and NR^{13R}R^{14R}, (2) C2-8 alkenyl which may be substituted by 1 to 3 substitutents selected from C1-10 alkyl, a halogen atom, hydroxyl, alkoxy, alkylthio, and NR^{13R}R^{14R}, (3) C2-8 alkynyl which may be substituted by 1 to 3 substitutents selected from C1-10 alkyl, a halogen atom, hydroxyl, alkoxy, alkylthio, and NR^{13R}R^{14R}, (4) C1-8 alkyl substituted by ring4^{R}, or (5) ring4^{R},
   R^{13R} and R^{14R} are each independently (1) a hydrogen atom, or (2) C1-10 alkyl,
   ring1^{R}, ring2^{R}, ring3^{R}, or ring4^{R} may be substituted by 1 to 5 R^{R},
   R^{R} is (1) C1-10 alkyl, (2) C2-10 alkenyl, (3) C2-10 alkynyl, (4) C1-10 alkoxy, (5) C1-10 alkylthio, (6) a halogen atom, (7) hydroxy, (8) nitro, (9) -NR^{15R}R^{16R}, (10) C1-10 alkyl substituted by C1-10 alkoxy, (11) C1-10 alkyl substituted by 1 to 3 halogen atoms, (12) C1-10 alkyl substituted by C1-10 alkoxy substituted by 1 to 3 halogen atoms, (13) C1-10 alkyl substituted by -NR^{15R}R^{16R}, (14) ring5^{R}, (15) -O-ring5^{R}-, (16) C1-10 alkyl substituted by ring5^{R}, (17) C2-10 alkenyl substituted by ring5^{R}, (18) C2-10 alkynyl substituted by ring 5^{R}, (19) C1-10 alkoxy substituted by ring5^{R}, (20) C1-10 alkyl substituted by -O-ring5^{R}, (21) COOR^{17R}, (22) C1-10 alkoxy substituted by 1 to 4 halogen atoms, (23) formyl, (24) C1-10 alkyl substituted by hydroxyl, or (25) C2-10 acyl,
   R^{15R}, R^{16R}, and R^{17R} are each independently (1) a hydrogen atom, or (2) C1-10 alkyl,
   ring5^{R} may be substituted by 1 to 3 substitutents selected from following (1) to (9);
      (1) C1-10 alkyl, (2) C2-10 alkenyl, (3) C2-10 alkynyl, (4) C1-10 alkoxy, (5) C1-10 alkyl substituted by C1-10 alkoxy, (6) a halogen atom, (7) hydroxyl, (8) C1-10 alkyl substituted by 1 to 3 halogen atoms, (9) C1-10 alkyl substituted by C1-10 alkoxy substituted by 1 to 3 halogen atoms;
   ring1^{R}, ring2^{R}, ring3^{R}, ring4^{R} and ring5^{R} are each independently, (1) C3 to 15 mono-, bi- or tri-carbocyclic aryl which may be partially or fully saturated or (2) 3 to 15 membered mono-, bi- or tri-heterocyclic aryl containing hetero atoms selected from 1 to 4 nitrogen atoms, 1 to 2 oxygen atoms and/or 1 to 2 sulfur atoms which may be partially or fully saturated wherein,
      1) when E^{R} is E^{2R}, E^{2R} is U^{1R}-U^{2R}-U^{3R}, and U^{1R} is C2 alkylene or C2 alkenylene, U^{2R} is not -CHOH-,
      2) when U^{3R} is C1-8 alkyl substituted by at least one hydroxyl, U^{1R}-U^{2R} is neither C2 alkylene nor C2 alkenylene,
      3) when A^{R} is A^{1R} and D^{R} is D^{1R}, E^{R} is not E^{1R},
      4) when T^{R} is oxygen atom, X^{R} is -CH₂-, D^{R} is D^{1R}, D^{1R} is COOH, A^{R} is A^{1R}, A^{1R} is strainght chain C2-8 alkylene, E^{R} is E^{2R} , E^{2R} is U^{1R}-U^{2R}-U^{3R}, U^{1R} is C1-4 alkylene, and U^{3R} is C1-8 alkyl, U^{2R} is neither bond, -CH₂-, NR^{12R}-, nor carbonyl,
      5) when T^{R} is oxygen atom, X^{R} is CH₂-, D^{R} is D^{1R}, D^{1R} is COOH, A^{R} is A^{2R}, G^{1R} is C1-4 alkylene, G^{2R} is -O- or -NR^{1R}-, G^{3R} is bond or C1-4 alkylene, E^{R} is E^{2R}, E^{2R} is U^{1R}-U^{2R}-U^{3R}, U^{1R} is C1-4 alkylene and U^{3R} is C1-8 alkyl, then U^{2R} is not bond, -CH₂-, -NR^{12R}-, or carbonyl,
      6) when T^{R} is oxygen atom, X^{R} is -CH₂-, D^{R} is D^{1R}, E^{R} is E^{2R}, E^{2R} is U^{1R}-U^{2R}-U^{3R}, U^{1R} is C2 alkylene or C2 alkenylene and U^{2R} is -CO-, then A^{R} is not A^{1R},
      7) 4-[(2-{(2R)-2-[(1E,3S)-3-hydroxy-oct-1-enyl]-5-oxo-pyrrolidin-1-yl}ethyl]thio]butanoic acid and 4-{2-[(R)-2-((E)-3-hydroxy-oct-1-enyl)-5-oxa-pyrrolidin-1-yl]-ethyl}-benzoic acid are excluded.),
         a salt thereof, an N-oxide thereof or a solvate thereof, or a prodrug thereof, or a cyclodextrin clathrate thereof.
(S) In the pamphlet of US2005/0049227, it is described that the compound represented by the following formula (IS) has EP₄ agonistic activity. As well, the definition of each group of the compound represented by formula (IS) is described in the pamphlet of US2005/0049227. Accordingly, EP₄ agonist of the present invention includes the compound represented by formula (IS): 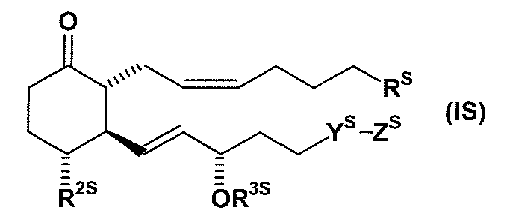 wherein R^{S} is CO₂R^{4S}, CONR^{4S}₂, CH₂OR^{4S}, CONR^{4S}SO₂R^{S4}, P(O)(OR^{4S}), 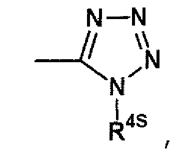
   R^{4S} is a hydrogen atom, phenyl, C1-6 alkyl,
   R^{1S} and R^{2S} are each independently a hydrogen atom, hydroxyl, C1-6 alkyloxy, C1-6 acyloxy,
   R³⁵ is a hydrogen atom, C1-6 alkyl, C1-6 acyl,
   Y^{S} is a bond, or -CH₂-, -O-, -S-, -N-,
   Z^{S} is C3-10 alkyl, C3-10 cycloalkyl, 6 to 10 membered aromatic carbocyclic ring, 4 to 10 membered aromatic heterocyclic ring containing one hetero atom selected from a nitrogen atom, an oxygen atom and a sulfur atom.,
      a salt thereof, an N-oxide thereof or a solvate thereof, or a prodrug thereof, or a cyclodextrin clathrate thereof.
(T) In the pamphlet of WO2004/085430, it is described that the compound represented by the following formula (IT) has EP₄ agonistic activity. As well, the definition of each group of the compound represented by formula (IT) is described in the pamphlet of WO2004/085430. Accordingly, EP₄ agonist of the present invention includes the compound represented by formula (IT): 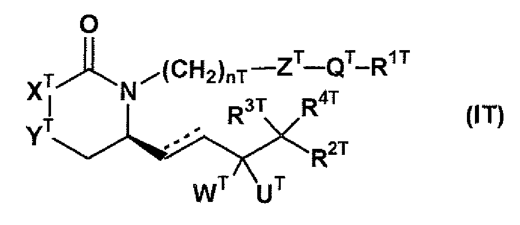 wherein, Q^{T} is (CH₂)_{mT}, (CH₂)_{mT}-C6-10 aryl, (CH₂)_{mT}-C5-10 heterocyclic ring, (CH₂)_{mT}-C3-10 heterocyclic alkyl, (CH₂)_{mT}-C3-8 cycloalkyl, methylene substituted by two halogen atoms, said cycloalkyl, heterocyclic alkyl, aryl, or heterocyclic ring may be substituted by three R_{aT},
   X^{T} and Y^{T} are each independently methylene, an oxygen atom, a nitrogen atom substituted by R^{9T}, a sulfur atom, provided that, X^{T} and Y^{T} are not an oxygen atom, a nitrogen atom substituted by R^{9T}, or a sulfur atom at the same time,
   U^{T} is a hydrogen atom, C1-3 alkyl, or is not present when W^{T} is oxo, W^{T} is hydroxyl, oxo, provided that U^{T} is not present when W^{T} is oxo, R^{1T} is -(CH₂)_{pT}-hydroxyl, -(CH₂)_{pT}-cyano,-(CH₂)_{pT}-CO₂R^{10T}, -(CH₂)_{nT}-SO₃R^{6T},-(CH₂)_{pT}-CF₂SO₂NH₂, -(CH₂)_{pT}-SO₂NH₂, -(CH₂)_{pT}-CONHSO₂R^{2T}, -(CH₂)_{pT}-SO₂NHCOR^{2T}, -(CH₂)_{pT}-PO(OH)₂, (CH₂)_{pT}-CONHPO₂R^{6T}, -(CH₂)_{pT}-CONHR^{8T},-(CH₂)_{pT}-C1-4 alkoxy, -(CH₂)_{pT}-cycloalkyl, -(CH₂)_{pT}-hydroxymethylketone,-(CH₂)_{nT}-heterocyclic ring, said heterocyclic ring optionally is substituted by 1 to 3 R^{aT} and may contain an acidic hydroxyl,
   R^{2T} is independently C1-10 alkyl, (CH₂)_{mT}-C6-10 aryl, (CH₂)_{mT}-C5-10 heterocyclic ring, (CH₂)_{mT}-C3-10 heterocyclic alkyl, (CH₂)_{mT}-C3-8 cycloalkyl, O-C1-10 alkyl, O-C6-10 aryl, O-C3-10 cycloalkyl, O-C3-10 heterocyclic alkyl, provided that R^{2T} is O-C1-10 alkyl, O-C6-10 aryl, O-C3-10 cycloalkyl, O-C3-10 heterocyclic alkyl, then R^{3T} and R^{4T} are not halogen atoms, said alkyl, cycloalkyl, heterocyclic alkyl, aryl, or heterocyclic ring which may be substituted by 1 to 3 R^{aT},
   R^{3T} and R^{4T} are each independently hydrogen atom, halogen atom, C1-6 alkyl, or R^{3T} and R^{4T} may be taken together to form a 3-7 membered carbocyclic ring which may contain of 1 to 2 hetero atoms selected from an oxygen atom, a sulfur atom, SO, SO₂ and a nitrogen atom substituted by R^{9T}
   R^{6T} and R^{7T} are each independently a hydrogen atom, C1-4 alkyl, R^{8T} is a hydrogen atom, acyl, sulfonyl,
   R^{9T} is a hydrogen atom, C1-6 alkyl, said alkyl may be substituted by 1 to 3 halogen atoms, cyano, hydroxyl, C1-6 alkoxy, C1-6 acyloxy, amino,
   R^{10T} is a hydrogen atom, C1-10 alkyl, C3-10 cycloalkyl, (CH₂)_{pT}-C6-10 aryl, (CH₂)_{pT}-C5-10 hetercylcic ring, CR^{6T}R^{7T}OC(O)-C3-10 cycloalkyl, CR^{6T}R^{7T}OC(O)-C1-10 alkyl,
   Z^{T} is a triple bond, an oxygen atom, sulfur atom, (C(R^{bT})₂)_{nT}, - CH=CH-,
   R^{bT} is a hydrogen atom, C1-6 alkyl, a halogen atom,
   R^{aT} is C1-6 alkoxy, C1-6 alkyl, CF₃, nitro, amino, cyano, C1-6 alkylamino, halogen atom, R^{aT} is further aryl, heterocyclic ring, S-C1-6 alkyl, S-C6-10 aryl, S-C5-10 heterocyclic ring, CO₂R^{6T}, O-C6-10 aryl, O-C5-10 heterocyclic ring, CH₂O-C1-6 alkyl, CH₂S-C1-6 alkyl, CH₂O-aryl, CH₂S-aryl,
   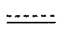 is a double bond or single bond,
   pT is 0 to 3,
   nT is 0 to 4,
   mT is 0 to 8.],
   a salt thereof, an N-oxide thereof or a solvate thereof, or a prodrug thereof, or a cyclodextrin clathrate thereof.
(U) In the pamphlet of WO2004/085431 it is described that the compound represented by the following formula (IU) has EP₄ agonistic activity. As well, the definition of each group of the compound represented by formula (IU) is described in the pamphlet of WO2004/085431. Accordingly, EP₄ agonist of the present invention includes the compound represented by formula (IU): 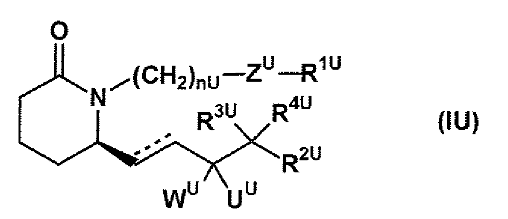 wherein U^{U} is a hydrogen atom, C1-3 alkyl, or is not present when W^{U} is oxo,
   W^{U} is hydroxyl, oxo, provided that, U^{U} is not present when W^{U} is oxo,
   Z^{U} is (CH₂)_{nU}, -CH=CH-,
   R^{1U} is (CH₂)_{pU}-hydroxyl, (CH₂)_{pU}-CO₂R^{10U}, (CH₂)_{nU} heterocyclic ring, said heterocyclic ring may be substituted by 1 to 3 R^{aU} and may contain an acidic hydroxyl,
   R^{2U} is independently C1-10 alkyl, (CH₂)_{mU}-C6-10 aryl, (CH₂)_{mU}-C5-10 heterocyclic ring, (CH₂)_{mU}-C3-10 heterocyclic alkyl, (CH₂)_{mU}-C3-8 cycloalkyl, said alkyl, cycloalkyl, heterocyclic alkyl, aryl, or a heterocyclic ring may be substituted by 1 to 3 R^{aU},
   R^{3U} and R^{4U} are each independently a hydrogen atom, a halogen atom, C1-6 alkyl,
   R^{6U} is a hydrogen atom, C1-4 alkyl,
   R^{10U} is a hydrogen atom, C1-10 alkyl, C3-10 cycloalkyl, (CH₂)_{pU}-C6-10 aryl, (CH₂)_{pU}-C5-10 heterocyclic ring,
   R^{aU} is C1-6 alkoxy, C1-6 alkyl, CF₃, nitro, amino, cyano, C1-6 alkylamino, halogen atom, R^{aU} is further aryl, a heterocyclic ring, S-C1-6 alkyl, S-C6-10 aryl, S-C5-10 heterocyclic ring, O-C6-10 aryl, O-C5-10 heterocyclic ring, CO₂R^{6U}, CH₂O-C1-6 alkyl, CH₂S-C1-6 alkyl, CH₂O-aryl CH₂S-aryl,
   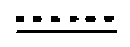 is a double bond or a single bond,
   pU is 0 to 3,
   nU is 0 to 4,
   mU is 0 to 8,
   a salt thereof, an N-oxide thereof or a solvate thereof, or a prodrug thereof, or a cyclodextrin clathrate thereof.
(W) In the pamphlet of W02004/063158, it is described that the compound represented by the following formula (IW) has EP₄ agonistic activity. As well, the definition of each group of the compound represented by formula (IW) is described in the pamphlet of WO2004/063158. Accordingly, EP₄ agonist of the present invention includes the compound represented by formula (IW): 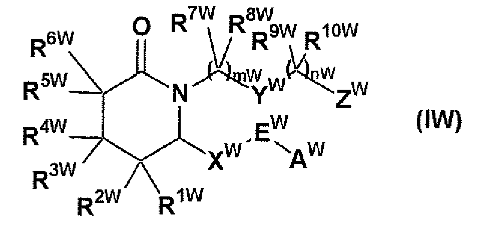 wherein, mW is 1 to 4,
   nW is 0 to 4,
   A^{W} is alkyl, aryl, heteroaryl, arylalkyl, arylcycloalkyl, cycloalkylalkyl, aryloxyalkyl,
   E^{W} is -CHOH-, -C(O)-,
   X^{w} is -(CH₂)₂-, -CH=CH-,
   Y^{W} is -CH₂-, -CH=CH-, arylene, heteroarylene, -O-, -S(O)_{pW}- wherein pW is 0 to 2, -NR^{aW}- wherein R^{aW} is a hydrogen atom, alkyl,
   Z^{W} is -CH₂OH-, -CHO, tetrazol-5-yl, -COOR^{bW} wherein R^{bw} is a hydrogen atom, alkyl,
   R^{1W}, R^{2W}, R^{3W}, R^{4W}, R^{5W}, R^{6W}, R^{7W}, R^{8W}, R^{9W} and R^{10W} are each independently a hydrogen atom, alkyl,
   a salt thereof, an N-oxide thereof or a solvate thereof, or a prodrug thereof, or a cyclodextrin clathrate thereof.

As EP₄ agonist of the present invention, the compound represented by formula (I): 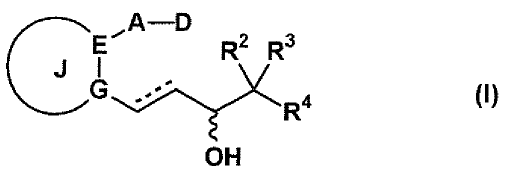 wherein all the symbols have the same meaning as the above described] is preferred, the compound represented by formula (IA) to (IW) more preferably is listed, the compound represented by formula (IA), (IB), (IQ) further preferably is listed.

As particularly preferably compound, a compound selected from
({3-[((1R,2S,3R)-3-hydroxy-2-{(1E,3S)-3-hydroxy-4-[3-(methoxymethyl)phenyl]but-1-enyl}-5-oxocyclopentyl)sulfanyl]propyl}sulfanyl)acetic acid;
4-{[2-((1R,2R,3R)-3-hydroxy-2-{(1E,3S)-3-hydroxy-4-[3-(methoxymethyl)phenyl]but-1-enyl}-5-oxocylcopentyl)ethyl]sulfanyl}butanoic acid;
7-((1R,2R,3R)-3-hydroxy-2-{(1E,3S)-3-hydroxy-4-[3-(methoxymethyl)phenyl]but-1-enyl}-5-oxocyclopentyl)heptanoic acid;
(5Z)-7-((1R,2R,3R)-2-{(1E,3S)-4-[3-(ethoxymethyl)phenyl]-3-hydroxybut-1-enyl}-3-hydroxy-5-oxocyclopentyl)hept-5-enoic acid;
(5Z)-7-((1R,2R,3R,5R)-5-chloro-2-{(1E,3S)-4-[3-(ethoxymethyl)phenyl]-3-hydroxybut-1-enyl}-3-hydroxycyclopentyl)hept-5-enoic acid;
4-[(2-{(1R,2R,3R)-3-hydroxy-2-[(1E,3S)-3-hydroxy-4-(4-hydroxy-3-methylphenyl)but-1-enyl]-5-oxocyclopentyl}ethyl)sulfanyl]butanoic acid;
methyl 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}-butanoate;
4-{[2-((1R,2R)-2-{(1E,3S)-3-hydroxy-4-[3-(methoxymethyl)phenyl]but-1-enyl}-5-oxocyclopentyl)ethyl]sulfanyl}butanoic acid;
4-[(2-{(2R)-2-[(1E,3S)-4-(3-chlorophenyl)-3-hydroxybut-1-enyl]-5-oxopyrrolidin-1-yl}ethyl)sulfanyl]butanoic acid;
4-{[2-((2R)-2-{(1E,3S)-3-hydroxy-4-[3-(trifluoromethyl)phenyl]but-1-enyl}-5-oxopyrrolidin-1-yl)ethyl]sulfanyl}butanoic acid;
4-[(2-{(2R)-2-[(1E,3S)-4-(4-fluorophenyl)-3-hydroxybut-1-enyl]-5-oxopyrrolidin-1-yl}ethyl)sulfanyl]butanoic acid;
4-[(2-{(2R)-2-[(1E,3S)-3-hydroxy-4-(2-naphthyl)-but-1-enyl]-5-oxopyrrolidin-1-yl}ethyl)sulfanyl]butanoic acid;
4-[(2-{(4S)-4-[(1E,3S)-4-(4-fluorophenyl)-3-hydroxy-1-butenyl]-2-oxo-1,3-oxazolidin-3-yl}ethyl)sulfanyl]butanoic acid;
2-[(2-{(2R)-2-[(1E,35)-3-hydroxy-4-(3-methylphenyl)but-1-enyl]-5-oxopyrrolidin-1-yl}ethyl)sulfanyl]-1,3-thiazol-4-carboxylic acid;
2-[(2-{(2R)-2-[(1E,3S)-3-hydroxyoct-1-enyl]-5-oxopyrrolidin-1-yl}ethyl)sulfanyl]-1,3-thiazol-4-carboxylic acid;
2-{[2-((2R)-2-{(1E,3S)-4-[3-(1-benzofuran-2-yl)phenyl]-3-hydroxybut-1-enyl}-5-oxopyrrolidin-1-yl)ethyl]sulfonyl}-1,3-thiazol-4-carboxylic acid;
4-[(2-{(2R)-2-[(1E(35)-3-hydroxyocat-1-enyl]-5-oxopyrrolidin-1-yl}ethyl)sulfanyl]butanoic acid;
{[3-({(1R,2S,3R)-3-hydroxy-2-E(1E,3S)-3-hydroxyoct-1-enyl]-5-oxocyclopentyl}sulfanyl)propyl]sulfanyl}acetic acid; and
2-[(2-{(4S)-4-[(1E,3S)-4-(4-fluorophenyl)-3-hydroxybut-1-enyl]-2-oxo-1,3-oxazolidin-3-yl}ethyl)sulfanyl]-1,3-thiazol-4-carboxylic acid,
a salt thereof, a hydrate thereof or a prodrug thereof, or a cyclodextrin clathrate thereof is listed. As most preferably compound, a compound selected from
({3-[((1R,2S,3R)-3-hydroxy-2-{(1E,3S)-3-hydroxy-4-[3-(methoxymethyl)phenyl]but-1-enyl}-5-oxocyclopentyl)sulfanyl]propyl}sulfanyl)acetic acid;
methyl 4-{[2-((1R,2R,3R)-3-hydroxy-2-{(1E,3S)-3-hydroxy-4-[3-(methoxymethyl)phenyl]but-1-enyl}-5-oxocyciopentyl)ethyl]sulfanyl}butanoate; and
4-[(2-{(2R)-2-[(1E,3S)-4-(4-fluorophenyl)-3-hydroxybut-1-enyl]-5-oxopyrrolidin-1-yl}ethyl)sulfanyl]butanoic acid,
a salt thereof, a hydrate thereof or a prodrug thereof, or a cyclodextrin clathrate thereof is listed.

### [Isomer]

Unless otherwise specified, all isomers are included in the present invention. For example, alkyl, alkenyl, alkynyl, alkyloxy, alkoxy, alkenyloxy, alkynyloxy, alkylthio, alkylsulfinyl, alkylsulfonyl, alkylene, alkenylene, alkynylene, acyl and acyloxy group means straight-chain or branched-chain ones. In addition, isomers on double bond, ring, fused ring (E-, Z-, cis-, trans-isomer), isomers generated from asymmetric carbon atom(s) (R-, S-isomer, α⁻, β-configuration, enantiomer, diastereomer), optically active isomers (D-, L-, d-, l-isomer), polar compounds generated by chromatographic separation (more polar compound, less polar compound), equilibrium compounds, rotational isomers, mixtures thereof at voluntary ratios and racemic mixtures are also included in the present invention.

According to the present invention, unless otherwise indicated and as is apparent for those skilled in the art, the symbol 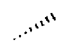 indicates that it is bound to the opposite side of the sheet (namely α-configuration), the symbol 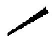 indicates that it is bound to the front side of the sheet (namely β-configuration), the 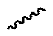 indicates that it is a α-configuration, β-configuration or a mixture thereof which may be mixed by optional ratio, and the symbol 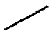 indicates that it is a mixture of α-configuration and β-configuration.

### [Salt and Solvate]

The compounds represented by formula (I) can be converted into salts thereof by known methods. As salts, non-toxic, water-soluble salts are preferred.

The salts of the present invention include for example, salts of alkali metals (*e.g*., potassium, sodium, lithium, *etc*.), salts of alkaline earth metals (*e.g.*, calcium, magnesium, *etc*.), ammonium salts (*e.g.*, tetramethylammonium salt, tetrabutylammonium salt, *etc*.), pharmaceutical acceptable salts of organic amine (*e.g.*, triethylamine, methylamine, dimethylamine, cyclopentylamine, benzylamine, phenethylamine, piperidine, monoethanolamine, diethanolamine, tris(hydroxymethyl)methylamine, lysine, arginine, N-methyl-D-glucamine, *etc*.), acid addition salts (salts of inorganic acids (*e.g*., hydrochloride, hydrobromide, hydroiodide, sulfate, phosphate, nitrate *etc*.), and salts of organic acids (*e.g.*, acetate, trifluoroacetate, lactate, tartrate, oxalate, fumarate, maleate, benzoate, citrate, methanesulfonate, ethanesulfonate, benzenesulfonate, toluenesulfonate, isethionate, glucuronate, gluconate *etc*.).

The compound of the present invention can be made to N-oxide by a voluntary method. N-oxide means nitrogen atom of the compound represented by formula (I) is oxidized.

The compound represented by formula (1), a salt thereof, or an N-oxide thereof can be also converted into a solvate. The solvate preferably includes non-toxic, soluble ones. A solvate of the present invention includes the solvate of, such as, water, alcohol solvent (*e.g*., methanol, ethanol *etc*.) and so on.

### [Cyclodextrin clathrate compound]

The compounds of the present invention represented by formula (I), a salt thereof, an N-oxide thereof or a solvate thereof, or a prodrug thereof may be converted into the corresponding cyclodextrin clathrates by the method described in the specification of JP-B-50-3362, 52-31404 or 61-52146 using α⁻, p- or γ-cyclodextrin or a mixture thereof. Converting into the corresponding cyclodextrin clathrates serves to increase the stability and solubility in water of the compounds, and therefore it is useful in the use for pharmaceuticals.

### [Prodrug]

The prodrug of the compounds of the present invention means a compound is converted into the compound represented by formula (I) by reaction with enzymes, gastric acids and so on within an organism.

The prodrug of the compound of the present invention include, when the compounds represented by formula (I) have amino, the prodrug is the compound the amino of which is acylated, alkylated, or phosphorylated (*e.g.*, the compound is that the amino of the compound of present invention is eicosanoated, alanylated, pentylaminocarbonylated, (5-methyl-2-oxo-1,3-dioxolan-4-yl)methoxycarbonylated, tetrahydrofuranated, pyrrolidylmethylated, pivaloyloxymethylated, acetoxymethylated, tert-butylated, *etc*.); when the compound of the present invention has hydroxy, the prodrug is the compound the hydroxy of which is acylated, alkylated, phosphorylated or borated (*e.g*., the compound is that the hydroxy of the compound of the present invention is acetylated, palmitoylated, propanoylated, pivaloylated, succinylated, fumarylated, alanylated, dimethylaminomethylcarbonylated *etc*.); when the compound of the present invention has carboxy, the prodrug is the compound the carboxyl of which is esterified or amidated (*e.g.*, the compound is that the carboxy of the compound of the present invention is ethylesterified, phenylesterified, carboxymethylesterified, dimethylaminomethylesterified, pivaloyloxymethylesterified, ethoxycarbonyloxyethylesterified, phthalidylesterified, (5-methyl-2-oxo-1,3-dioxolen-4-yl)methylesterified, cyclohexyloxycarbonylethylesterified, methylamidated *etc*.); and so on.

These compounds can be prepared by known methods. In addition, the prodrug of the compound of the present invention may be either hydrate or non-hydrate. In addition, the prodrug of the compound of the present invention may be converted into the compound of the present invention under the physiological condition which is described in "the development of medicine" vol.7 "molecular design" published in 1991 Hirokawa shoten p.p. 163-198. Further, the compound represented by formula (I) may be labeled with isotopes (*e.g.* ³H, ¹⁴C, ³⁵S, ¹²⁵I *etc*.) and so on.

The compound in the present invention represented by formula (I), a salt thereof, an N-oxide thereof, or a solvate thereof, or a prodrug thereof or a cyclodextrin clathrate (hereinafter collectively abbreviated to the compound in the present invention) is the compound which has superior solubility, absorbability and metabolic stability, and sustains pharmacological activity (EP₄ agonistic activity) for a long time, additionally, has weak inhibitory activity of drug-metabolizing enzyme and lower toxicity, such as actions on circulatory organs and so on. These properties are most important physical, chemical, pharmaceutical properties which are required in case of development of pharmaceutical products. The compound in the present invention meets these conditions and has a possibility to be great pharmaceutical products. [refer to The Merck Manual of Diagnosis and Therapy (17th Ed.), Merck & Co.]

It can be evaluated that the compound in the present invention is useful for pharmaceutical product by the methods described in the following various experimental systems, biological examples and the methods which can be properly improved them to practice. In addition, it can be easily evaluated that the compound in the present invention is superior pharmacokinetically in respect of, such as, length of serum half-life, stability in gastrointestinal tract, oral absorbability, bioavailability *etc*., by the known method, such as, the methods described in "Drug bioavailability (Science of evaluation and improvement)", Gendai-Iryo, published in 1998/07/06 and so on.

### [Processes for the preparation of the compound in the present invention]

The compound in the present invention can be prepared by known methods, such as, the methods described in WO03/009872, WO00/003980, EP855389, EP985663, WO00/015608, WO01/149661, WO01/166518, PCT/JP2004/000419 (WO2004/065365), WO01/24647, WO02/042268, WO03/008377, WO03/035064, WO03/053923, WO03/103664, WO03/007941, US2005/0049227, WO04/085430, WO04/085431 or WO04/063158, or the pursuant methods thereof.

### [Toxicity]

Toxicity of the compound in the present invention is very low, and it is safe enough to use as a pharmaceutical agent.

### [Application to pharmaceutical products]

Since EP₄ agonist promotes potassium excretion, it is useful as a preventive and/or therapeutic agent for hyperkalemia. In particular, it is useful as a preventive and/or therapeutic agent for hyperkalemia in the patients who have renal failure or abnormality of renal function. In addition, since EP₄ agonist is useful as a preventive and/or therapeutic agent for hyperkalemia, it is useful for ameliorating various symptoms of hyperkalemia, e.g., (1) neural and/or muscular disorder such as, pareshtesia, error of perception, weakness, myoparalysis (e.g., dyspnea caused by diaphragmatic paralysis, asphyxia *etc*.) and the like, (2) gastrointestinal dysfunction such as, nausea, vomit, abdominal pain, diarrhea and the like, (3) circulatory dysfunction such as, feeble heart sound, electrocardiogram change, arrhythmia, ventricular fibrillation, atrial fibrillation, atrioventicular block, cardiac arrest and the like.

EP₄ agonist which is a preventive and/or therapeutic agent for hyperkalemia of the present invention may be administered in combination with other pharmaceutical preparations to accomplish the following purposes:
1) To compensate for and/or enhance the preventive and/or treatment effect of hyperkalemia;
2) To improve the kinetics/absorption of the compound to be combined and reduce the dose of said EP₄ agonist of the present invention; and/or
3) To eliminate the side effect of said EP₄ agonist of the present invention to be combined

The concomitant medication of EP₄ agonist of the present invention and other pharmaceutical preparations may be administered in the form of formulation having these components incorporated in one preparation or may be administered in separate preparations. In the case where these pharmaceutical preparations are administered in separate preparations, they may be administered simultaneously or at different times. In the case of administration at different times, EP₄ agonist of the present invention may be administered before the other pharmaceutical preparations. Alternatively, the other pharmaceutical preparations may be administered before EP₄ agonist of the present invention. Each method for the administration of these pharmaceutical preparations may be the same or different.

The other pharmaceutical preparations may be low-molecular compounds. In addition, they may be macromolecular protein, polypeptide, polynucleotide (DNA, RNA, and gene), antisense, decoy, antibody or vaccine and so on. The dose of the other pharmaceutical preparations can be accordingly selected as a standard of clinical dose. Additionally, the compounding ratio of EP₄ agonist of the present invention and the other pharmaceutical preparations can be accordingly selected by the age and body weight of administering object, the administration method, the administration time, *etc*. For example, the other pharmaceutical preparations may be used from 0.01 to 100 parts by weight relative to 1 part by weight of EP₄ agonist of the present invention. The other pharmaceutical preparations may be administered at appropriate ratio combining one or more arbitrarily. The other pharmaceutical preparations to compensate for and/or enhance the preventive and/or treatment effect of hyperkalemia do not only include ones which have ever been found but ones which will be found from now based on the above-mentioned mechanism.

The other pharmaceutical preparations include, for example, calcium formulation, glucose formulation, insulin formulation, sodium bicarbonate formulation, diuretic agent (e.g. mannitol, furosemide, acetazolamide, diclofenamide, methazolamide, trichloromethiazide, mefruside, aminophylline *etc*.) and cation exchange resin (e.g. polystyrene sulfonate sodium, polystyrene sulfonate calcium *etc*.) and so on.

In order to use EP₄ agonist of the present invention or EP₄ agonist of the present invention in combination with the other medicaments, these are normally administered to the entire or local part of human body orally or parenterally.

The doses to be administered are differently determined depending upon, for example, age, body weight, symptom, the desired therapeutic effect, the route of administration, and the duration of the treatment *etc*. In the human adult, the doses per person are generally from 0.1 ng to 1000 mg, by oral administration, from one time to several times per day, and from 0.1 ng to 100 mg, by parenteral administration, from one time to several times per day, or continuous administration from 1 to 24 hours per day from vein.

As mentioned above, the doses vary depending upon various conditions. Therefore, there are cases in which doses lower than the above described doses are enough or more administration is necessary greater doses than the ranges specified above.

EP₄ agonist of the present invention, or concomitant medication combined EP₄ agonist of the present invention with other preparations may be administered in the composition of, for example, solid compositions or liquid compositions, each for oral administration, or injections, external use, suppositories, eye drops inhalant each for parenteral administration.

Examples of the solid preparations for internal use for oral administration include tablets, pills, capsules, powders, granules and the like. The capsules include hard capsules and soft capsules. The tablets include sublingual tablets, intraoral patches, orally fast disintegrating tablets and the like.

Such a solid preparation for internal use is prepared by a formulation method commonly employed by using one or two or more active substances either as it is or as a mixture with an excipient (lactose, mannitol, glucose, microcrystalline cellulose, starch, *etc*.), a binder (hydroxypropylcellulose, polyvinylpyrrolidone, magnesium metasilicate aluminate, *etc*.), a disintegrating agent (calcium cellulose glycolate, *etc*.), a lubricant (magnesium stearate, *etc*.), a stabilizer and a dissolution aid (glutamic acid, aspartic acid, *etc*.). If necessary, it may be coated with a coating agent (sucrose, gelatin, hydroxypropylcellulose, hydroxypropylmethylcellulose phthalate, *etc*.). It may be coated with two or more layers. Moreover, capsules made of an absorbable material such as gelatin are involved in the scope thereof.

The sublingual tablets may be prepared in accordance with a well known method. For example, a sublingual tablet is prepared by a formulation method commonly employed by using one or more active substances mixed with an excipient (lactose, mannitol, glucose, microcrystalline cellulose, colloidal silica, starch, *etc*.), a binder (hydroxypropylcellulose, polyvinylpyrrolidone, magnesium metasilicate aluminate, *etc*.), a disintegrator (starch, L-hydroxypropyl cellulose, carboxymethyl cellulose, croscarmellose sodium, calcium cellulose glycolate, *etc*.), a lubricant (magnesium stearate, *etc*.), a swelling agent (hydroxypropyl cellulose, hydroxylpropylmethy cellulose, carbopol, carboxymethyl cellulose, polyvinyl alcohol, xanthan gum, guar gum, e*tc.*), a swelling aid agent (glucose, fructose, mannitol, xylitol, erythritol, maltose, trehalose, phosphate, citrate, silicate, glycine, glutamic acid, arginine, *etc*.), a stabilizer, a dissolution aid (polyethylene glycol, propylene glycol, glutamic acid, aspartic acid, *etc*.), a flavoring agent (orange, strawberry, mint, lemon, vanilla, *etc*.). If necessary, it may be coated with a coating agent (sucrose, gelatin, hydroxypropylcellulose, hydroxypropylmethylcellulose phthalate, *etc.*). If necessary, it may be coated with two or more layers. Moreover, it may also further comprise some additives such as antiseptics, antioxidants, coloring agents, sweetening agents and the like.

The intraoral patch may be prepared in accordance with a well known method. For example, a intraoral patch is prepared by a formulation method commonly employed by using one or more active substances mixed with an excipient (lactose, mannitol, glucose, microcrystalline cellulose, colloidal silica, starch, *etc*.), a binder (hydroxypropylcellulose, polyvinylpyrrolidone, magnesium metasilicate aluminate, *etc*.), a disintegrator (starch, L-hydroxypropyl cellulose, carboxymethyl cellulose, croscarmellose sodium, calcium cellulose glycolate, *etc.*)*,* a lubricant (magnesium stearate, *etc*.), a attach agent (hydroxypropyl cellulose, hydroxylpropylmethy cellulose, carbopol, carboxymethyl cellulose, polyvinyl alcohol, xanthan gum, guar gum, *etc*.), a attach aid agent (glucose, fructose, mannitol, xylitol, erythritol, maltose, trehalose, phosphate, citrate, silicate, glycine, glutamic acid, arginine, *etc*.), a stabilizer, a dissolution aid (polyethylene glycol, propylene glycol, glutamic acid, aspartic acid, *etc.*)*,* a flavoring agent (orange, strawberry, mint, lemon, vanilla, *etc.*) and the like. If necessary, it may be coated with a coating agent (sucrose, gelatin, hydroxypropylcellulose, hydroxypropylmethylcellulose phthalate, *etc*.) and the like. If necessary, it may be coated with two or more layers. Moreover, it may also further comprise some additives such as antiseptics, antioxidants, coloring agents, sweetening agents and the like.

The orally fast disintegrating tablet may be prepared in accordance with a well known method. For example, a orally fast disintegrating tablet is prepared by a formulation method commonly employed by using one or more active substances directly, or active substances by covering with adequate coating agent (ethylcellulose, hydroxypropycellulose, hydroxypropylmethylcellulose, acrylic acid and methacrylic acid copolymer *etc*.), plasticizer (polyethylenegrycol, triethyl citrate *etc*.) to bulks or granulating bulk particles mixed with an excipient (lactose, mannitol, glucose, microcrystalline cellulose, colloidal silica, starch, *etc*.), a binder (hydroxypropylcellulose, polyvinyl pyrrolidone, magnesium metasilicate aluminate, *etc*.), a disintegrator (starch, L-hydroxypropyl cellulose, carboxymethyl cellulose, croscarmellose sodium, calcium cellulose glycolate, *etc.*), a lubricant (magnesium stearate, *etc*.), a dispersion aid agent (glucose, fructose, mannitol, xylitol, erythritol, maltose, trehalose, phosphate, citrate, silicate, grylcine, glutamate, arginine *etc*.), a stabilizer and a dissolution aid (polyethylene glycol, propylene glycol, glutamic acid, aspartic acid, *etc*.), a flavoring agent (orange, strawberry, mint, lemon, vanilla, *etc*.) and the like. If necessary, it may be coated with a coating agent (sucrose, gelatin, hydroxypropylcellulose, hydroxypropylmethylcellulose phthalate, *etc*.) and the like. If necessary, it may be coated with two or more layers. Moreover, it may also further comprise some additives such as antiseptics, antioxidants, coloring agents, sweetening agents and the like.

Liquid forms for oral administration include pharmaceutically acceptable solutions, suspensions and emulsions, syrups and elixirs. In such forms, one or more of the active compound(s) may be dissolved, suspended or emulized into diluent(s) commonly used in the art (such as purified water, ethanol or a mixture thereof). Besides such liquid forms may also comprise some additives, such as wetting agents, suspending agents, emulsifying agents, sweetening agents, flavoring agents, aroma, preservative or buffering agent.

In the parenteral administration, formulation of external use include, for example, ointment, gel, cream, poultice, patch, liniment, atomized agent, inhalation, spray, aerosol, eye drops and nasal spray, *etc*. They includes one or more of the active compound(s) and be prepared by known method or usual method.

Ointment is prepared by known method or usual method. For example, it is prepared by levigation or fusion of one or more of the active compound(s) and substrate. The substrate of ointment is selected from known or usual one. For example, higher fatty acid or higher fatty acid ester (adipic acid, myristic acid, palmitic acid, stearic acid, oleic acid, adipic acid ester, myristic acid ester, palmitic acid ester, stearic acid ester, oleic acid ester, *etc*.), wax (yellow beeswax, spermaceti, ceresin, *etc*.), surfactant (polyoxyethylene alkyl ether phosphoric acid ester, *etc*.), higher alcohol (cetanol, stearil alcohol, cetostearyl alcohol, *etc.*), silicon oil (dimethyl polysiloxane, *etc*.), hydrocarbon (hydrophilic petrolatum, white petrolatum, purified lanolin, liquid paraffin, *etc*.), glycol (ethylene glycol, diethylene glycol, propylene glycol, polyethylene glycol, macrogol, *etc*.), vegetable oil (castor oil, olive oil, sesame oil, turpentine oil, *etc*.), animal oil (mink oil, egg yolk oil, squalane, squalene, *etc*.), water, absorption accelerator, skin fit inhibitor, *etc*. are used as single substance selected from them or mixture which consists of two or more kinds that is selected from them. Moreover, humectant, preservative agent, stabilizer, antioxidative agent, fragrant materials, *etc.* may be contained.

Gel is prepared by known method or usual method. For example, it is prepared by fusion of one or more of the active compound(s) and substrate. The substrate of gel is selected from known or usual one. For example, lower alcohol (ethanol, isopropylalcohol, *etc.*)*,* gelling agent (carboxy methyl cellulose, hydroxy ethyl cellulose, hydroxy propyl cellulose, ethyl cellulose, *etc*.), neutralizing agent, (triethanolamine, diisopropanolamine, *etc*.), surfactant, (polyethylene glycol monostearate, *etc*.), gum, water, absorption accelerator, skin fit inhibitor, *etc*. are used as single substance selected from them or mixture which consists of two or more kinds that is selected from them. Moreover, preservative agent, antioxidative agent, fragrant materials, *etc*. may be contained.

Cream is prepared by known method or usual method. For example, it is prepared by fusion or emulsification of one or more of the active compound(s) and substrate. The substrate of cream is selected from known or usual one. For example, higher fatty acid ester, lower alcohol, hydrocarbon, polyalcohol (propylene glycol, 1,3-butylene glycol, *etc*.), higher alcohol (2-hexyldecanol, cetanol, *etc.*)*,* emulsifying agent (polyoxyethylene alkyl ether, fatty acid ester, *etc*.), water, absorption accelerator, skin fit inhibitor, *etc*. are used as single substance selected from them or mixture which consists of two or more kinds that is selected from them. Moreover, preservative agent, antioxidative agent, fragrant materials, *etc*. may be contained.

Poultice is prepared by known method or usual method. For example, it is prepared by fusion of one or more of the active compound(s) and substrate, and then the kneaded one is laid over support medium. The substrate for poultice is selected from known or usual one. For example, thickening agent (polyacrylic acid, polyvinylpyrolidone, gum acacia, starch, gelatin, methyl cellulose, *etc*.), a wetting agent (urea, glycerin, propylenegrycol *etc*.), bulking agent (kaolin, zinc oxide, talc, calcium, magnesium, *etc*.), water, solubilizing agent, thickener, skin fit inhibitor, *etc*. are used as single substance selected from them or mixture which consists of two or more kinds that is selected from them. Moreover, preservative agent, antioxidative agent, fragrant materials, *etc.* may be contained. Patch is prepared by known method or usual method. For example, it is prepared by fusion of one or more of the active compound(s) and substrate, and then laid over support medium. The substrate for patch is selected from known or usual one. For example, polymer substrate, fat, higher fatty acid, thickener, skin fit inhibitor, *etc*. are used as single substance selected from them or mixture which consists of two or more kinds that is selected from them. Moreover, preservative agent, antioxidative agent, fragrant materials, *etc*. may be contained.

Liniment is prepared by known method or usual method. For example, one or more of the active compound(s) may be dissolved, suspended or emulsified in water, alcohol (ethanol, polyethylene glycol, *etc*.), higher fatty acid, glycerin, soap, emulsifying agent, suspending agent, *etc*. as single substance selected from them or mixture which consists of two or more kinds that is selected from them. Moreover, preservative agent, antioxidative agent, fragrant materials, *etc*. may be contained.

Atomized agent, inhalation and spray may comprise in addition to a diluent, a stabilizer such as sodium bisulfite and an isotonization buffer such as sodium chloride, sodium citrate or citric acid. The preparation process of sprays is described in detail in, for example, U.S. Patent Nos. 2,868,691 and 3,095,355.

The dosage of inhalations for parenreral administration include aerosol, powders for inhalation or liquids for inhalation. The liquids for inhalation may be dissolved or suspended in water or the other appropriate solvent as needed.

Such inhalations are prepared in a known method.

For example, a liquid for inhalation is prepared by selecting proper additives from an antiseptic (benzalkonium chloride or p-aminobenzonic acid), a coloring agent, a buffering agent (sodium phosphate or sodium acetate), an isotonizing agent (sodium chloride or concentrated glycerin), thickening agent (carboxyvinylpolymer), or an accelerator of absorption, *etc.,* if necessary. A powder for inhalation is prepared by selecting proper additives from a lubricant agent (such as stearin acid and the salt thereof), a binding agent, (such as starch, dextrin), a diluting agent (such as lactose, cellulose), a coloring agent, an antiseptic (such as benzalkonium chloride or p-aminobenzonic acid), an accelerator of absorption, *etc*., if necessary.

In case of administration of liquid for inhalation, spray (atomizer, nebulizer) is usually used and in case of administration of powder for inhalation, inhalation administration apparatus for powder agents is usually used.

Injections for parenteral administration include sterile aqueous, suspensions, emulsions and solid forms which are dissolved or suspended into solvent(s) for injection immediately before use. In injections, one or more of the active compound(s) may be dissolved, suspended or emulized into solvent(s), The solvents may include distilled water for injection, physiological salt solution, vegetable oil, propylene glycol, polyethylene glycol, alcohol, e.g. ethanol, or a mixture thereof, Injections may comprise some additives, such as stabilizing agents, solution adjuvants (glutamic acid, aspartic acid, POLYSORBATE80 (registered trade mark) *etc*.), suspending agents, emulsifying agents, soothing agent, buffering agents, preservative. They may be sterilized at a final step, or may be prepared by an aseptic manipulation. They may also be manufactured in the form of sterile solid forms, for example, freeze-dried products, which may be dissolved in sterile water or some other sterile diluent(s) for injection immediately before use.

The other compositions for parenteral administration include suppositories for intrarectal administration and pessaries for vaginal administration which comprise one or more of the active substance(s) and may be prepared by methods known per se.

In addition, in case of administration to dialysis patients the above described injections can be also administered at dialysis.

### [Effect of the Invention]

Since EP₄ agonist promotes potassium excretion, it is useful as a preventive and/or therapeutic agent for hyperkalemia. Further, since EP₄ agonist does not cause chlorine ion secretion if selective EP₄ agonist uses, it is a preventive and/or therapeutic agent for hyperkalemia without side effects such as, diarrhea *etc*., so that it is useful.

### [Brief Description of the Drawing]

Figure 1 is a figure showing the change of short-circuit current (I*sc*) by the addition of PGE₂.
Figure 2 is a graph showing the relation between variation (ΔI*sc*) of short-circuit current (*Isc*) and PGE₂ in each, as the change of short-circuit current (I*sc*) by the addition of PGE₂ divides to transient phase and sustained phase.
Figure 3 is a figure showing the change of short-circuit current (I*sc*) in case of addition of compound 1 (({3-[((1R,2S,3R)-3-hydroxy-2-{(1E,3S)-3-hydroxy-4-[3-(methoxymethyl)phenyl]but-1-enyl)-5-oxocyclopentyl)sulfanyl] propyl}sulfanyl)acetic acid).
Figure 4 is a graph making a comparison between variation (ΔI*sc*) of short-circuit current (I*sc*) in case of addition of compound 1 in transient phase and variation (ΔI*sc*) of short-circuit current (I*sc*) in case of addition of PGE₂ in transient phase.
Figure 5 is a graph making a comparison between variation (ΔI*sc*) of short-circuit current (I*sc*) in case of addition of compound 1 in sustained phase and variation (ΔI*sc*) of short-circuit current (I*sc*) in case of addition of PGE₂ in sustained phase.

### [Best Mode for Carrying Out the Invention]

It is proved by the following experiments that EP₄ agonist shows the effect of the present invention. As well, the present invention is not limited thereto.

### [Biological Examples]

### Example1: Measurement system for colonic ion transport in guinea pig using Ussing flux chamber

### (1-T.) Tissue sample preparation

Male Hartley-Hazleton guinea pigs (ranging in weight from 400 to 800g; Nippon SLC) were allowed water and food ad libitum, were stunned and then exsanguinated. After laparotomy, distal colon was removed, tissues were flushed with Krebs-Ringer solution (117.0mM NaCl, 4.7mM KCl, 1.2mM MgCl₂, 1.2mM NaH₂PO₄, 25.0mM NaHCO₃, 2.5mM CaCl₂, 11.0mM glucose), and cut along the mesenteric border. The incised colons were fixed by pins on Petri dish covered with silicon rubber with the mucosal side down. Tissue samples including submucosal ganglia and mucosa were obtained by removal of myenteric plexus, longitudinal muscle layer and circular muscle layer using scissors for ophthalmology under stereoscopic microscope with adding ice cold Krebs-Ringer solution into Petri dish.

### (1-2) Establishment of short-circuit current measurement system using Ussing flux chamber

Tissue samples prepared in Example 1-1 were mounted Ussing flux chambers in which the cross-sectional area was 0.64cm². Each chamber of mucosal and serosal surfaces of the tissues was filled with Krebs-Ringer solution (pH 7,2 - 7.4, 10mL) gassed with mixed gas (O₂:CO₂=95:5). As well, each chamber was together with thermostatic chamber to form the circuits. Krebs-Ringer solution warmed at 37°C was constantly circulated there during the experiment.

The potential difference (PD) between mucosal side and serosal side was measured by paired Ag-AgCl electrodes using Krebs-agar bridge. PD was adjusted to OmV by applying a short-circuit current (I*sc*) by further paired Ag-AgCl electrodes with a voltage-clamp apparatus (SS-1335; Nihon-Kohden Co.). Tissue conductance (*G*₁) was calculated by determining the current necessary to change PD by 10 mV. As well, measurement of a short-circuit current was performed using a chart recorder (Recti-Horitz-8K; Nihon-Denki Sanei) and Mac/Lab8 system (AD Instruments).

### (1-3) Pretreatment

To eliminate the influence of nerve cell and the effect of endogenous PG synthesis, tetrodotoxin (10⁻⁶ mol/L) added into serosal side chamber (hereinafter, abbreviated to serosal chamber), and after 10 minutes, piroxicam (10⁻⁵mol/L) added into the same chamber. It was confirmed that a short-circuit current (I*sc*) reached to plateau and then the following experiments were performed.

### (1-4) Investigation of EP₄ agonistic activity

EP₄ agonistic activity was measured by measurement of the change of short-circuit current (I*sc*) for 10 minutes after some various concentrations of EP₄ agonist added into serosal chamber.

As well, PGE₂ was used as control and ({3-[((1R,2S,3R)-3-hydroxy-2-{(1E,3S)-3-hydroxy-4-[3-(methoxymethyl)phenyl]but-1-enyl}-5-oxocyclopentyl)sulfanyl]propyl}sulfany)acetic acid (hereinafter abbreviated to compound 1) was used as EP₄ agonist.

### [Result]

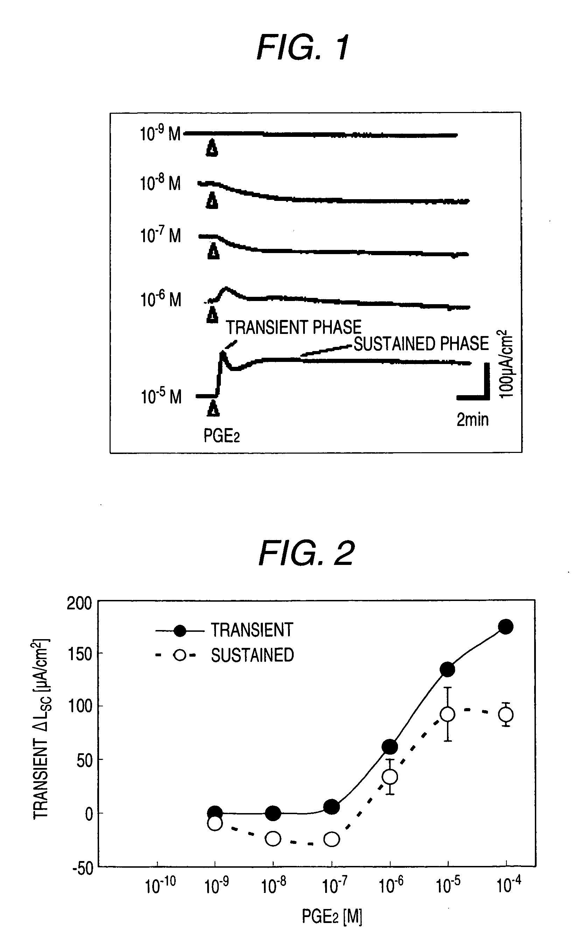

The change of short-circuit current (I*sc*) by PGE₂ addition showed in Figure 1. PGE₂ promoted the decrease of I*sc* at from 10⁻⁸ to 10⁻⁷ mol/L, the temporary increase of I*sc* at 10⁻⁶ mol/L, and the biphasic [Transient phase, Sustained Phase] change of short-circuit current (I*sc*) at 10⁻⁵ mol/L. Since the decrease of short-circuit current (I*sc*) suggested the potassium secretion, the result that PGE₂ promoted the potassium secretion at low concentration was obtained.

This change of short-circuit current (I*sc*) was calculated as variation (ΔI*sc*) by finding the difference between I*sc* after PGE₂ addition and I*sc* before PGE₂ addition. Figure 2 showed the relation between ΔI*sc* and PGE₂ concentration.

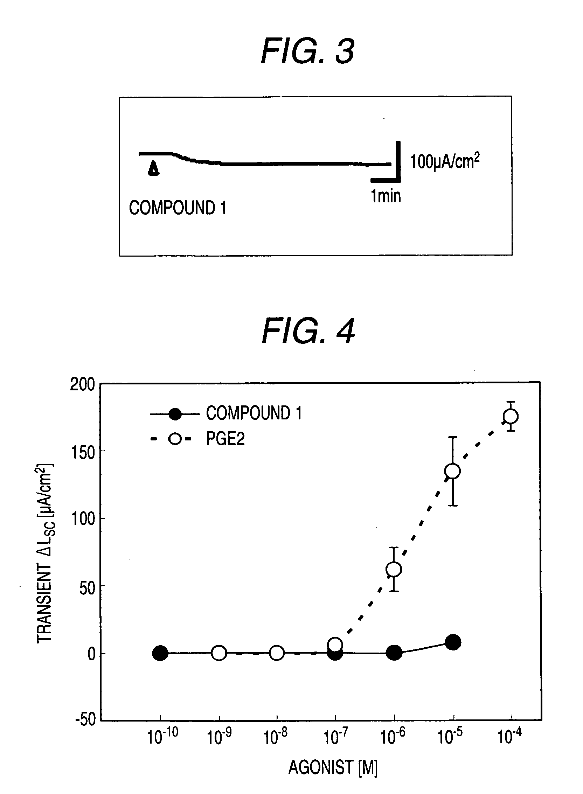

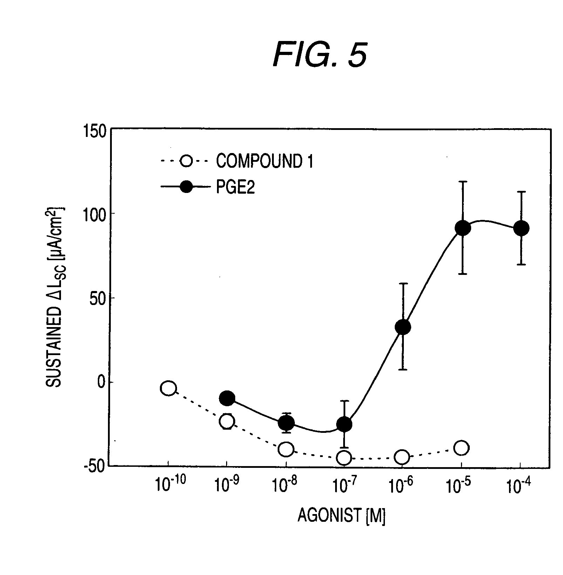

Similarly, figure 3 showed the change of short-circuit current (Isc) in case of the compound 1, EP₄ agonist, addition, figure 4 showed the relation between the variation (ΔI*sc*) in the transient phase and the concentration of the compound 1 and PGE₂, and figure 5 showed the relation between the variation (ΔI*sc*) in the sustained phase and the concentration of the compound 1 and PGE₂.

Since it was observed that the decrease of short-circuit current (I*sc*) by addition of test compound, EP₄ agonist, as well as by addition of PGE₂ at low concentration, it was found that EP₄ agonist had the action which promoted potassium secretion.

As well, it was confirmed that the decrease of short-circuit current (I*sc*) by PGE₂ at low concentration was countered by EP₄ antagonist. As a result, this action promoting potassium excretion was not only found in this compound, but also it was expected whatever compounds had EP4 agonistic action, had this action.

### Formulation Example 1: Tablet

A solution (100ml) of ({3-[((1R,2S,3R)-3-hydroxy-2-{(1E,3S)-3-hydroxy-4-[3-(methoxymethyl)phenyl]but-1-enyl}-5-oxocyclopentyl)sulfanyl]propyl}sulfany)acetic acid (30mg), magnesium stearate (1000mg), silicon dioxide (200mg), talc (100mg), and carboxymethylcellulose calcium (2000mg) were admixed by conventional method, dried and then micro crystalline cellulose (50.0g) added into the mixture and the total volume was adjusted to 100g. They were sufficiently admixed until they were equalized, and then punched out by conventional method to obtain 1000 tablets each containing 30µg of active ingredient.

### Formulation Example 2: Injection

α-cyclodextrin clathrate compound (60mg) of ({3-[((1R,2S,3R)-3-hydroxy-2-{(1E,3S)-3-hydroxy-4-[3-(methoxymethyl)phenyl]but-1-enyl}-5-oxocyclopentyl)sulfanyl]propyl}sulfany)acetic acid (5mg) was dissolved into distilled water for injection (3000ml), the solution was aseptic filtrated with membrane filter and then the solution 3ml each was filled into a 5ml capacity ampoule for injection to obtain injections (1000 ampoules) containing 5µg of active ingredient.

### Industrial applicability

EP₄ agonist is useful in the following as pharmaceutical product. For example, since EP₄ agonist promotes potassium excretion, it is useful as a preventive and/or therapeutic agent for hyperkalemia. In addition, if selective EP₄ agonist uses, it is a preventive and/or therapeutic agent for hyperkalemia without side effects, so that it is useful, Further, if EP₄ agonist is used, it is useful as improving agent for various symptoms (e.g. paresthesia, error of perception, weakness, myoparalysis, nausea, vomit, abdominal pain, diarrhea, arrhythmia, atrioventricular block, ventricular fibrillation, atrial fibrillation, cardiac arrest, and/or asphyxia *etc*.). Since EP₄ agonist promotes potassium excretion, it is useful for the patients who have renal damage and cannot excrete of potassium in the urine (particularly, renal failure patients *etc*.).

## Claims

1. A preventive and/or therapeutic agent for hyperkalemia, comprising EP₄ agonist.

2. The preventive and/or therapeutic agent according to claim 1, wherein one or more symptom(s) selected from nerve abnormality, muscular abnormality, circulatory abnormality and digestive abnormality is/are i m proved .

3. The preventive and/or therapeutic agent according to claim 1, wherein one or more symptom(s) selected from arrhythmia, atrioventricular block, ventricular fibrillation, atrial fibrillation and dyspnoea is/are improved.

4. A potassium excretion promoter, comprising EP₄ agonist.

5. The agent according to claim 1 or 4, wherein the EP₄ agonist and one or more agent(s) selected from calcium formulation, glucose formulation, insulin formulation, sodium bicarbonate formulation, diuretic, and cation exchange resin are used in combination.

6. The agent according to claim 1 or 4, wherein the EP₄ agonist is a compound represented by formula (I): 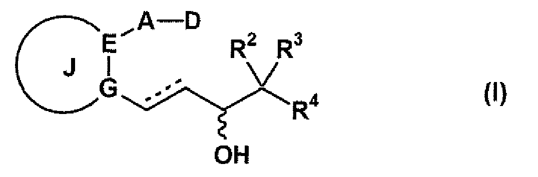 wherein 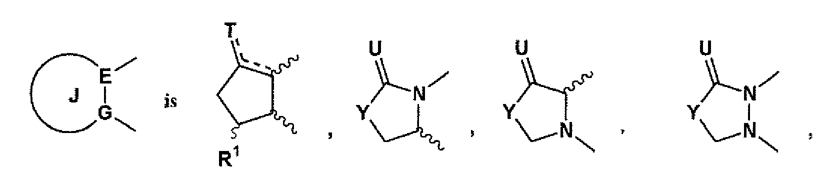 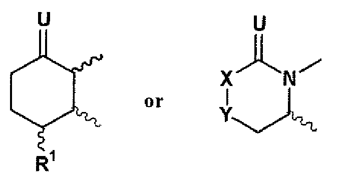 wherein T is an oxygen atom, a halogen atom or acyloxy which may have a substituent(s),
R¹ is a hydrogen atom, hydroxy, C1-6 alkyloxy or C1-6 acyloxy,
U is an oxygen atom or a sulfur atom,
X and Y are each independently methylene, an oxygen atom, a sulfur atom, or a nitrogen atom which may have a substituent(s), wherein X and Y are not an oxygen atom, a sulfur atom nor a nitrogen atom which may have a substituent(s) simultaneously,
A is a spacer of which main chain has an atom number of 1-8 and which may have a substituent(s),
D is an acidic group which may be protected,
R² and R³ are each independently alkyl which may have a substituent(s) or a halogen atom,
R⁴ is a cyclic group which may have a substituent(s) or aliphatic hydrocarbon which may have a substituent(s),
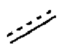 is a single bond or a double bond, wherein continuous double bonds are not formed,
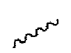 is α-configuration or β-configuration or a mixture thereof having an optical mixing ratio,
a salt thereof, an N-oxide thereof or a solvate thereof, or a prodrug thereof, or a cyclodextrin clathrate thereof.

7. The agent according to claim 1 or 4, wherein the EP₄ agonist is a compound represented by formula (IA): 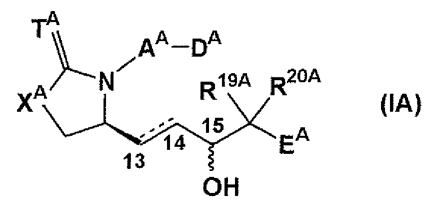 wherein 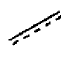 is (1) a single bond or (2) a double bond,
R^{19A} and R^{20A}are each independently, (1) a hydrogen atom, (2) C1-10 alkyl or (3) a halogen atom,
T^{A} is (1) an oxygen atom or (2) a sulfur atom,
X^{A} is (1) -CH₂-, (2) -O- or (3) -S-,
A^{A} is A^{1A} or A^{2A},
A^{1A} is (1) C2-8 straight-chain alkylene which may be substituted by 1 to 2 C1-4 alkyl, (2) C2-8 straight-chain alkenylene which may be substituted by 1 to 2 C1-4 alkyl or (3) C2-8 straight-chain alkynylene which may be substituted by 1 to 2 C1-4 alkyl,
A^{2A} is -G^{1A} -G^{2A} -G^{3A}
A^{2A} is -G^{1A} -G^{2A} -G^{3A} - -,
G^{1A} is (1) C1-4 straight-chain alkylene which may be substituted by 1 to 2 C1-4 alkyl, (2) C2-4 straight-chain alkenylene which may be substituted by 1 to 2 C1-4 alkyl or (3) C2-4 straight-chain alkynylene which may be substituted by 1 to 2 C1-4 alkyl,
G^{2A} is (1) -Y^{A} -, (2) -(ring1^{A})-, (3) -Y^{A}-(ring1^{A})-, (4) -(ring1^{A})-Y^{A}- or (5) -Y^{A}-(C1-4 alkylene)-(ring1^{A})-,
Y^{A} is (1) -S-, (2) -SO-, (3) -SO₂-, (4) -O- or (5) -NR^{1A}-,
R^{1A} is (1) a hydrogen atom, (2) C1-10 alkyl or (3) C2-10 acyl,
G^{3A} is (1) a bond, (2) C1-4 straight-chain alkylene which may be substituted by 1 to 2 C1-4 alkyl, (3) C2-4 straight-chain alkenylene which may be substituted by 1 to 2 C1-4 alkyl or (4) C2-4 straight-chain alkynylene which may be substituted by 1 to 2 C1-4 alkyl,
D^{A} is D^{1A} or D^{2A},
D^{1A} is (1) -COOH, (2) -COOR^{2A}, (3) tetrazol-5-yl or (4) CONR^{3A}SO₂R^{4A},
R^{2A} is (1) C1-10 alkyl, (2) phenyl, (3) C1-10 alkyl substituted by phenyl or (4) biphenyl,
R^{3A} is (1) a hydrogen atom or (2) C1-10 alkyl,
R^{4A} is (1) C1-10 alkyl or (2) phenyl,
D^{2A} is (1) -CH₂OH, (2) -CH₂OR^{5A}, (3) hydroxy, (4) -OR^{5A}, (5) formyl, (6) -CONR^{6A}R^{7A}, (7) -CONR^{6A}SO₂R^{8A}, (8) -CO-(NH-amino acid residue-CO)_{mA} -OH, (9) -O-(CO- amino acid residue -NH)_{mA} -H, (10) -COOR^{9A}, (11) -OCO-R^{10A}, (12) -COO-Z^{1A} -Z^{2A} -Z^{3A}, (13) 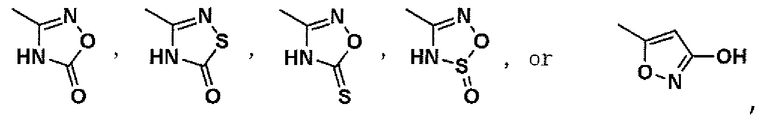
R^{5A} is C1-10 alkyl,
R^{6A} and R^{7A} are each independently, (1) a hydrogen atom or (2) C1-10 alkyl,
R^{8A} is C1-10 alkyl substituted by phenyl,
R^{9A} is (1) C1-10 alkyl substituted by biphenyl which may be
substituted by 1 to 3 C1-10 alkyl, C1-10 alkoxy or a halogen atom or (2) biphenyl substituted by 1 to 3 C1-10 alkyl, C1-10 alkoxy or a halogen atom,
R^{10A} is (1) phenyl or (2) C1-10 alkyl,
mA is 1 or 2,
Z^{1A} is (1) C1-15 alkylene, (2) C2-15 alkenylene or (3) C2-15 alkynylene,
Z^{2A} is (1) -CO-, (2) -OCO-, (3) -COO-, (4) -CONR^{11A}-, (5) -NR^{12A}CO-, (6) -O-, (7) -S-, (8) -SO-, (9) -SO₂-, (10) -NR^{13A}-, (11) -NR^{14A}CONR^{15A}-, (12) -NR^{16A}COO-, (13) -OCONR^{17A}- or (14) -OCOO-,
Z^{3A} is (1) a hydrogen atom, (2) C1-15 alkyl, (3) C2-15 alkenyl, (4) C2-15 alkynyl, (5) ring2^{A} or (6) C1-10 alkyl substituted by C1-10 alkoxy, C1-10 alkylthio, C1-10 alkyl-NR^{18A}- or ring2^{A},
R^{11A}, R^{12A}, R^{13A}, R^{14A}, R^{15A}, R^{16A} R^{17A} and R^{18A} are each independently (1) a hydrogen atom or (2) C1-15 alkyl,
R^{11A} and Z^{3A} may be taken together with the nitrogen atom to which they are attached to form a 5- to 7-membered saturated mono-heterocyclic ring, and the heterocyclic ring may contain other one hetero atom selected from oxygen, nitrogen and sulfur atom(s),
E^{A} is E^{1A} or E^{2A},
E^{1A} is (1) C3-7 cycloalkyl or (2) ring3^{A},
E^{2A} is (1) C3-7 cycloalkyl, (2) ring4^{A} or (3) ring5 ^{A},
ring1^{A} and ring5^{A} which may be substituted by 1 to 3 R^{21A} and/or R^{22A},
ring3^{A} may be substituted by 1 to 2 R^{21A},
C3-7 cycloalkyl represented by E^{2A} is substituted by one of R^{21A} or R^{22A}, and may be substituted by another 1 to 2 R^{21A} and/or R^{22A},
ring4^{A} is substituted by one of R^{22A}, may be substituted by another 1 to 2 R^{21A} and/or R^{22A}, and may be substituted by a heterocyclic ring formed by R^{11A},
Z^{3A} and the nitrogen to which Z^{3A} is attached or
ring2^{A} may be substituted by R^{23A},
R^{21A} is (1) C1-10 alkyl, (2) C1-10 alkoxy, (3) a halogen atom, (4) nitro, (5) C1-10 alkyl substituted by 1 to 3 halogen atom(s) or (6) phenyl,
R^{22A} is (1) C2-10 alkenyl, (2) C2-10 alkynyl, (3) C1-10 alkylthio, (4) hydroxy, (5) -NR^{24A}R^{25A}, (6) C1-10 alkyl substituted by C1-10 alkoxy, (7) C1-10 alkyl substituted by C1-10 alkoxy substituted by 1 to 3 halogen atom(s), (8) C1-10 alkyl substituted by -NR^{24A}R^{25A}, (9) ring6^{A}, (10) -O-ring7^{A}, (11) C1-10 alkyl substituted by ring7^{A}, (12) C2-10 alkenyl substituted by ring7^{A}, (13) C2-10 alkynyl substituted by ring7^{A}, (14) C1-10 alkoxy substituted by ring7 ^{A}, (15) C1-10 alkyl substituted by -O-ring7^{A}, (16) -COOR^{26A} or (17) C1-10 alkoxy substituted by 1 to 3 halogen atom(s),
R^{24A}, R^{25A} and R^{26A} are each independently, (1) a hydrogen atom or (2) C1-10 alkyl,
R^{23A} is (1) C1-15 alkyl, (2) C2-15 alkenyl, (3) C2-15 alkynyl or (4) C1-10 alkyl substituted by C1-10 alkoxy, C1-10 alkylthio or C1-10 alkyl-N R^{27A}-, ,
R^{27A} is (1) a hydrogen atom or (2) C1-10 alkyl,
ring1^{A}, ring2^{A}, ring5^{A}, ring6^{A} and ring7^{A} are (1) C3-15 mono-, bi- or tri-carbocyclic aryl which may be partially or fully saturated or (2) 3- to 15-membered mono-, bi- or tri-heterocyclic aryl containing 1 to 4 hetero atom(s) selected from oxygen, nitrogen and sulfur atom(s) which may be partially or fully saturated,
ring3^{A} and ring4^{A} are (1) thienyl, (2) phenyl or (3) furyl,
ring6^{A} and ring7^{A} may be substituted by 1 to 3 R^{28A},
R^{28A} is (1) C1-10 alkyl, (2) C2-10 alkenyl, (3) C2-10 alkynyl, (4) C1-10 alkoxy, (5) C1-10 alkyl substituted by C1-10 alkoxy, (6) a halogen atom, (7) hydroxy, (8) C1-10 alkyl substituted by 1 to 3 halogen atom(s) or (9) C1-10 alkyl substituted by C1-10 alkoxy substituted by 1 to 3 halogen atom(s), and wherein (1) when T^{A} is an oxygen atom, X^{A} is CH₂-, A^{A} is A^{1A}, and D^{A} is D^{1A}, E^{A} is E^{2A}, (2) ring5^{A} is not C3-7 cycloalkyl, phenyl, thienyl nor furyl, (3) when ring6^{A} is phenyl,the phenyl has at least one R^{28A},
a salt thereof, an N-oxide thereof or a solvate thereof, or a prodrug thereof, or a cyclodextrin clathrate thereof.

8. The agent according to claim 1 or 4, wherein the EP₄ agonist is a compound represented by formula (IB): 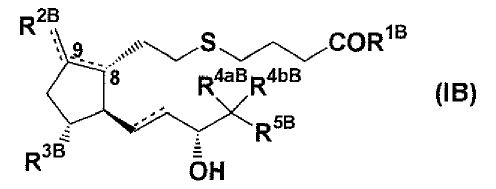 wherein R^{1B} is hydroxy, C1-6 alkyloxy or NR^{6B} R^{7B} in which R^{6B} and R^{7B} are each independently hydrogen or C1-4 alkyl,
R^{2B} is an oxygen atom, a halogen atom or O-COR^{8B} in which R^{8B} is C1-4 alkyl, phenyl or phenyl(C1-4 alkyl),
R^{3B} is a hydrogen atom or hydroxy,
R^{4aB} and R^{4bB} are each independently a hydrogen atom or C1-4 alkyl,
R^{5B} is phenyl substituted with the following substituent(s):
i) 1 to 3 selected from (a) C1-4 alkyloxy-C1-4 alkyl, (b) C2-4 alkenyloxy-C1-4alkyl, (c) C2-4 alkynyloxy-C1-4 alkyl, (d) C3-7 cycloalkyloxy-C1-4alkyl, (e) C3-7 cycloalkyl(C1-4 alkyloxy)-C1-4 alkyl, (f) phenyloxy-C1-4 alkyl, (g) phenyl-C1-4 alkyloxy-C1-4 alkyl, (h) C1-4 alkylthio-C1-4 alkyl, (i) C2-4 alkenylthio-C1-4 alkyl, (j) C2-4 alkynylthio-C1-4 alkyl, (k) C3-7 cycloalkylthio-C1-4 alkyl, (1) C3-7 cycloalkyl(C1-4 alkylthio)-C1-4 alkyl, (m) phenylthio-C1-4alkyl and (n) phenyl-C1-4 alkylthio-C1-4alkyl,
ii) (a) C1-4 alkyloxy-C1-4 alkyl and C1-4 alkyl, (b) C1-4 alkyloxy-C1-4 alkyl and C1-4 alkyloxy, (c) C1-4 alkyloxy-C1-4 alkyl and hydroxy, (d) C1-4 alkyloxy-C1-4 alkyl and halogen atom, (e) C1-4 alkylthio-C1-4 alkyl and C1-4 alkyl, (f) C1-4 alkylthio-C1-4 alkyl and C1-4 alkyloxy, (g) C1-4 alkylthio-C1-4 alkyl and hydroxy or (h) C1-4 alkylthio-C1-4 alkyl and a halogen atom,
iii) (a) haloalkyl or (b) hydroxy-C1-4 alkyl, or
iv) C1-4 alkyl and hydroxy;
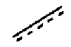 is a single bond or a double bond, wherein continuous double bonds are not formed, whrein when R^{2B} is O-COR^{8B}, the 8-9 position represents a double bond,
a salt thereof, an N-oxide thereof or a solvate thereof, or a prodrug thereof, or a cyclodextrin clathrate thereof.

9. The agent according to claim 1 or 4, wherein the EP₄ agonist is a compound selected from
({3-[((1R,2S,3R)-3-hydroxy-2-{(1E,3S)-3-hydroxy-4-[3-(methoxymethyl)phenyl]but-1-enyl}-5-oxocyclopentyl)sulfanyl]propyl}sulfanyl)acetic acid;
4-{[2-((1R,2R,3R)-3-hydroxy-2-{(1E,35)-3-hydroxy-4-[3-(methoxymethyl)phenyl]but-1-enyl}-5-oxocylcopentyl)ethyl]sulfanyl}butanoic acid;
7-((1R,2R,3R)-3-hydroxy-2-{(1E,3S)-3-hydroxy-4-[3-(methoxymethyl)phenyl]but- 1-enyl}-5-oxocydopentyl)heptanoic acid;
(5Z)-7-((1R,2R,3R)-2-{(1E,3S)-4-[3-(ethoxymethyl)phenyl]-3-hydroxybut-1-enyl}-3-hydroxy-5-oxocyclopentyl)hept-5-enoic acid;
(5Z)-7-((1R,2R,3R,SR)-5-chloro-2-{(1E,3S)-4-[3-(ethoxymethyl)phenyl]-3-hydroxybut-1-enyl}-3-hydroxycyclopentyl)hept-5-enoic acid;
4-[(2-{(1R,2R,3R)-3-hydroxy-2-[(1E,3S)-3-hydroxy-4-(4-hydroxy-3-methylphenyl)but-1-enyl]-5-oxncyclopentyl}ethyl)sulfanyl]butanoic acid;
methyl 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoate;
4-{[2-((1R,2R)-2-{(1E,3S)-3-hydroxy-4-[3-(methoxymethyl)phenyl]but-1-enyl}-5-oxocyclopentyl)ethyl]sulfanyl}butanoic acid;
4-[(2-{(2R)-2-[(1E,3S)-4-(3-chlorophenyl)-3-hydroxybut-1-enyl]-5-oxopyrrolidin-1-yl}ethyl)sulfanyl]butanoic acid;
4-{[2-((2R)-2-{(1E,35)-3-hydroxy-4-[3-(trifluoromethyl)phenyl]but-1-enyl}-5-oxopyrrolidin-1-yl)ethyl]sulfanyl}butanoic acid;
4-[(2-{(2R)-2-[(1E,3S)-4-(4-fluorophenyl)-3-hydroxybut-1-enyl]-5-oxopyrrolidin-1-yl}ethyl)sulfanyl]butanoic acid;
4-[(2-{(2R)-2-[(1E,3S)-3-hydroxy-4-(2-naphthyl)-but-1-enyl]-5-oxopyrrolidin-1-yl}ethyl)sulfanyl]butanoic acid;
4-[(2-{(4S)-4-[(1E,3S)-4-(4-fluorophenyl)-3-hydroxy-1-butenyl]-2-oxo-1,3-oxazolidin-3-yl}ethyl)sulfanyl]butanoic acid;
2-[(2-{(2R)-2-[(1E,3S)-3-hydroxy-4-(3-methylphenyl)but-1-enyl]-5-oxopyrrolidin-1-yl}ethyl)suffanyl]-1,3-thiazol-4-carboxylic acid;
2-[(2-{(2R)-2-[(1E,3S)-3-hydroxyoct-1-enyl]-5-oxopyrrolidin-1-yl}ethyl)sulfanyl]-1,3-thiazol-4-carboxylic acid;
2-{[2-((2R)-2-{(1E,3S)-4-[3-(1-benzofuran-2-yl)phenyl]-3-hydroxybut-1-enyl}-5-oxopyrrolidin-1-yl)ethyl]sulfanyl}-1,3-thiazol-4-carboxylic acid;
4-[(2-{(2R)-2-[(1E,3S)-3-hydroxyocat-1-enyl]-5-oxopyrrolidin-1-yl}ethyl)sulfanyl]butanoic acid;
{[3-({(1R,2S,3R)-3-hydroxy-2-[(1E,3S)-3-hydroxyoct-1-enyl]-5-oxocyclopentyl}sulfanyl)propyl]sulfanyl}acetic acid; and
2-[(2-{(4S)-4-[(1E,3S)-4-(4-fluorophenyl)-3-hydroxybut-1-enyl]-2-oxo-1,3-oxazolidin-3-yl}ethyl)sulfanyl]-1,3-thiazol-4-carboxylic acid,
a salt thereof, an N-oxide thereof or a solvate thereof, or a prodrug thereof, or a cyclodextrin clathrate thereof.

10. A method for preventing and/or treating hyperkalemia which comprises administering an effective amount of EP₄ agonist to a mammal.

11. A method for promotion of potassium excretion which comprises administering an effective amount of EP₄ agonist to a mammal.

12. Use of EP₄ agonist for preparing a preventive and/or therapeutic agent for hyperkalemia.

13. Use of EP₄ agonist for preparing a potassium excretion promoter.
